# EUROPEAN PATENT SPECIFICATION

(11) **EP 2 523 673 B1**
(45) Date of publication and mention of the grant of the patent: **03.05.2017**
(21) Application number: 11709193.4
(22) Date of filing: 04.01.2011
(51) Int. Cl.: A61K 35/74, A61Q 19/08, C12R 1/25, C07K 14/335, C12N 1/20, C12P 21/02, C12P 39/00, C12R 1/225, A61K 38/16

(54) **PROCESS FOR THE PREPARATION OF A BIOMASS COMPRISING PLANTARICIN AND USES THEREOF IN MEDICAL FIELD**
VERFAHREN ZUR HERSTELLUNG EINER BIOMASSE MIT PLANTARICIN UND DEREN VERWENDUNG IN DER MEDIZIN
PROCÉDÉ POUR LA PRÉPARATION D'UNE BIOMASSE COMPRENANT DE LA PLANTARICINE ET UTILISATIONS DE CELUI-CI DANS LE DOMAINE MÉDICAL

(30) Priority: 12.01.2010 IT RM20100004
(43) Date of publication of application: 21.11.2012
(73) Proprietor: GIULIANI S.p.A., I-20129 Milano (IT)
(72) Inventor: GIULIANI Giammaria, 20129 Milano (IT); BENEDUSI Anna, 20129 Milano (IT); GOBBETTI Marco, 70125 Bari (IT); Dl CAGNO Raffaella, 20129 Milano (IT); DE ANGELIS Maria, 70125 Bari (IT); CALASSO Maria, 70125 Bari (IT)
(74) Representative: Gitto, Serena
(86) International application number: PCT/IT2011/000003
(87) International publication number: WO 2011/086589

(56) References cited:
- EP-A1- 2 050 434
- WO-A1-2006/000992
- US-A1- 2005 196 480
- MALDONADO ANTONIO ET AL: "Production of plantaricin NC8 by Lactobacillus plantarum NC8 is induced in the presence of different types of gram-positive bacteria.", ARCHIVES OF MICROBIOLOGY, vol. 181, no. 1, January 2004 (2004-01), pages 8-16, XP002591121, ISSN: 0302-8933 cited in the application
- MALDONADO ANTONIO ET AL: "Induction of plantaricin production in Lactobacillus plantarum NC8 after coculture with specific gram-positive bacteria is mediated by an autoinduction mechanism.", JOURNAL OF BACTERIOLOGY, vol. 186, no. 5, March 2004 (2004-03), pages 1556-1564, XP002591122, ISSN: 0021-9193
- PARENTE E ET AL: "Production, recovery and purification of bacteriocins from lactic acid bacteria", APPLIED MICROBIOLOGY AND BIOTECHNOLOGY, vol. 52, no. 5, November 1999 (1999-11), pages 628-638, XP002591123, ISSN: 0175-7598
- DZUNG BAO DIEP ET AL: "A BACTERIOCIN-LIKE PEPTIDE INDUCES BACTERIOCIN SYNTHESIS IN LACTOBACILLUS PLANTARUM C11", MOLECULAR MICROBIOLOGY, vol. 18, no. 4, 1 January 1995 (1995-01-01), pages 631-639, XP009016839, WILEY-BLACKWELL PUBLISHING LTD, GB ISSN: 0950-382X, DOI: 10.1111/J.1365-2958.1995.MMI_18040631.x
- DE VUYST LUC ET AL: "The biodiversity of lactic acid bacteria in Greek traditional wheat sourdoughs is reflected in both composition and metabolite formation.", APPLIED AND ENVIRONMENTAL MICROBIOLOGY, vol. 68, no. 12, December 2002 (2002-12), pages 6059-6069, XP002591124, ISSN: 0099-2240

## Description

The present invention concerns a process for preparation of biomass comprising plantaricin and uses thereof in medical field. In particular, the present invention concerns a process for preparation of plantaricin A, N or K or mixtures thereof or a biomass containing one or more of above mentioned plantaricins in association with lactic acid bacteria used for preparation and uses thereof in order to stimulate the barrier function of intestinal cells or human epidermal keratinocytes.

It is known that epidermis and intestinal wall carry out a barrier function against external environment and harmful agents therein. Epidermis and internal wall barrier function can be weakened due to various factors. One of the conditions more frequently resulting in alterations of epidermis barrier function is the exposure to variously originating environmental factors. Under normal conditions anyway the epidermis is able to adapt to possible exogenous injuries through an adaptation resulting in a "steady state" achievement and adequate tolerance grade. Continuous aggression of the epidermis by irritating detergents and chemical substances (organic solvents, soaps, detergent solutions), for example, can damage not only the lipid component of epidermis surface film, but also the intercellular one of corneous layer resulting in barrier function degradation.

This results in low grade dermatitis characterized by an increase of water trans-epidermal loss, minor desquamation and elasticity loss of corneous layer, possibly followed by the formation of small continuous surface solutions. This mild irritating condition, often sub-clinical, triggers restoration processes that, through an increase of lipid synthesis and stimulation of basal keratinocyte proliferation activity allows new equilibrium and barrier function restoration to be achieved.

When dermal barrier is no more suitably to carry out adequately intrinsic defensive functions, the risk for onset of inflammatory cutaneous pathologies, triggered by cytokine release, resulting in the production in situ of flogogenic mediators and free radicals, increases. The latter, in addition to generation of direct DNA and protein injuries by oxidative mechanism, can cause the peroxidation of dermal cellular membranes.

Although epidermis does not contain blood vessels, anyway contains a small water amount indispensable for physiological equilibrium and corneous tissue integrity. Water from dermal capillaries flows through the dermal-epidermal junction and channel among corneocyte rows up to levels similar to most tissues, i.e. about 60-70% in the deep zones (compact corneous), and 10-35% in more superficial regions (disjunct corneous). The fact that, also in more external part, in contact with air, the epidermis is able to maintain a moisture reservoir is based on two particular functions: the barrier activity preventing the evaporation of fluid and the global hydrophilic activity of corneous layer (water holding capacity), resulting from the presence of high hygroscopic particles. Experiments based on progressive stripping of thinnest corneocyte layers of disjunct corneous layer, and hydrolipid overlapping film, demonstrate that barrier function is not remarkably affected, while, on the other hand, the same is gradually degraded by the removal of deeper layers of underlying compact corneous layers, whose cemented wall structure, with corneocyte bricks consisting of flaggrin compacted keratin clusters and rigid protein envelope is well known. By electron microscopy it has been shown that corneocytes are tightly sealed by modified desmosomial plates (corneosomes) and are embedded within a lipid adhesive known as intercorneocyte concrete, generating a film of flexible and nearly impenetrable barrier. Stressful conditions possibly affect acutely or chronically the cutaneous barrier and against the same the body generates a series of homeostatic mechanisms resulting in the capacity of the epidermis to monitor and restore the efficiency when failure thereof occurs independently on the origin of injurious acute noxa. The epidermis responds to chronic cutaneous stresses, induced by an extended exposure to low moisture of air, as typically in dry climates it occurs, through compensative adaptive phenomena resulting in proliferation increase of basal cells and consequent thickness increase of corneous layer and epidermis as a whole. Also the production and exocytosis of Odland bodies and intercorneocyte lipids (maintaining usual composition) increase so as the epidermis can retain water resources in a better way. In any case moisture deficiency results in a phase change as to the liquid-lipid stratification of the intercorneocyte concrete with subsequent crystallization. As a result lesser plasticity and rigidity as to tractions induced by the muscle-articular movements and extrinsic dynamic stimuli occur, thus easy microlesions are generated. Another implication of similar adaptive responses consists of hyperkeratosis: the proliferation layer increase is not counterbalanced by a corresponding higher exfoliation rate of corneocytes that, rather than to be scaled, remain embedded in corneous massive clusters and are detached only as large scales. Moreover, chronic environmental stresses activate a cytokine cascade with phlogosis histological aspects in association with hypertrophy and degranulation of derma mastocytes. This can explain the exacerbation of inflammatory dermatopathologies as observed in winter.

Every acute injury against epidermis barrier induces a cytokine response by involved cutaneous cells, the effects thereof are reflected on keratinocytes and underlying derma, resulting in important morphologic and functional consequences aiming to the restoration of the barrier function, epidermal surface texturing, and, above all, stimulation of fibroblasts activity on which the improvement of derma compactness and turgor is based on.

Actually the methods used in order to restore the barrier function are based on the use of restoring and hydroregulating substances suitable to obviate to insufficient cutaneous defense resulting from the alteration of the barrier function. These substances are compounds with re-hydrating and restructuring activity. Many dermocosmetic products allow the cutaneous re-hydration and according to restoring function thereof, favour cutaneous regeneration. However the same act through a simple "cosmetic masking" of a rough surface and do not possess any true therapeutic effect, that would have to exert through a repairing and regenerative activity aiming to the restoration of cutaneous morphofunctional integrity.

It has been recently outlined that bacteria are suitable to release and detect signal molecules as a response to environmental condition modifications, including variations of cellular density thereof and/or number of other microbial cell species occurring within an ecosystem (Sturme et al., 2007. Making sense of quorum sensing in lactobacilli: a special focus on Lactobacillus plantarum WCFA1. Microbilogy 153: 3939-3947). As to lactic acid bacteria, these responses, occurring according to a "quorum sensing" (QS) mechanism, include signal molecules named type 2 (Al-2, mainly furanones derivatives) auto-inducers, synthesized using LuxS enzyme activity (Miller e Bassler, 2003. LuxS quorum sensing: more than just a numbers game. Curr Opin Microbiol 6: 191-197), or signal molecules named peptide pheromone or peptide auto-inducer (AIP)(Nakajama et al., 2001. Gelatinase biosynthesis-activating pheromone: a peptide lactone that mediates a quorum sensing in Enterococcus faecalis. Mol Microbiol 41: 145-154). It has been recently proved that *L. plantarum* WCFS1 genome contains high number of genes encoding for AIP peptides, together with other genes encoding for other functions involved in "quorum sensing" mechanisms (Sturme et al., 2007. Making sense of quorum sensing in lactobacilli: a special focus on Lactobacillus plantarum WCFA1. Microbilogy 153: 3939-3947). Some studies have proved that the system aiming to the synthesis of plantaricin type peptide pheromone is involved in intra-species cellular communication mechanisms. In this case the peptide pheromone is used as a means to measure the cell density of molecule synthesizing species (Diep et al., 1994. The gene encoding plantaricin A, a bacteriocin from Lactobacillus plantarum C11, is located on the same transcription unit as an agr-like regulatory system. Appl Environ Microbiol 60:160-166). Other studies have also proved that plantaricin type peptide pheromones can be involved in mechanisms of inter-species cell communication. Particularly, the presence of competitive microorganisms can activate the regulating system involved in mechanisms of microbial antagonism (Maldonado et al., 2004. Production of plantaricin NC8 by Lactobacillus plantarum NC8 is induced in the presence of different types of Gram-positive bacteria. Arch Microbiol 181: 8-16). In the presence of other microbial species at cell high density, the peptide pheromone favours a cascade series of phosphorylation reactions involving metabolic regulation complex phenomena resulting in the synthesis of signal molecules specifically acting as bacteriocin type antimicrobial compounds (for example plantaricins A, K and N) (Hauge et al., 1998. Plantaricin A is an ampkiphilic alpha-helical bacteriocin-like pheromone which exerts antimicrobial and pheromone activities through different mechanisms. Biochemistry 37:16026-16032).

Although the mechanism of cell communication among prokaryotic and eukaryotic cells has been partially elucidated, very limited literature exists as to interactions among signal molecules involved in "quorum sensing" mechanisms of bacteria (for example. peptide pheromones) and cells of human intestinal mucosa. The unique example is CSF pentapeptide, synthesized by *Bacillus subtilis* probiotic microorganism, as a molecule involved in competence and sporulation phenomena (Fujija et al., 2007. The Bacillus subtilis quorum-sensing molecule CSF contributes to intestinal homeostasis via OCTN2, a host cell membrane transporter. Cell Host Microbe 1:299-308). It has been demonstrated that said pentapeptide is suitable to induce p38 MAP kinase, B kinase (Akt) and cryotolerance, thus favouring the prevention of oxidative damage at intestinal level and reinforcing barrier function. Further at current state of the art no publication or patent focused on the effect of signal molecules involved in mechanisms of bacteria cellular communication with the respect to human epidermis exists.

The Authors of the present invention now have discovered that plantaricin, particularly plantaricin A, exerts a positive effect on barrier function of keratinocytes in human epidermis and intestinal cells as well.

Studies about processes for plantaricin preparation using bacteria mono-cultures (intra-species) are known. However, the culture of the interest microorganisms is carried out in complex and too expensive culture media to be scaled up at industrial level for the preparation of signal molecules for therapeutic purpose.

The Authors of the present invention now have developed a process for the preparation of plantaricin using a co-culture of two specific lactic acid bacteria suitable to obtain a higher yield than those obtained according to known art. Particularly the cultivation of *L. plantarum* DC400 (deposited at DSMZ on 21 December 2009 with number DSM 23213) as a co-culture with *L. rossiae* DPPMA174 (deposited at DSMZ on 21 December 2009 with number DSM 23214) is suitable to activate the synthesis of plantaricin type peptide pheromone (particularly plantaricin A) obtaining concentrations about 50 fold higher than in the presence of *L*. *plantarum* DC400 monoculture (DSM 23213). Further it has been proved that *L. plantarum* DC400 (DSM 23213) culture with other species of lactic acid bacteria, also isolated by "natural sourdough", is not suitable to stimulate the synthesis of peptide pheromone as when in association with *L. rossiae.* Another important aspect of the process according to the present invention is that the synthesis of plantaricin A is viable not only in culture media usually employed for lactic acid bacteria laboratory culture, but also on grape must, milk serum and aqueous extracts from fruit and vegetable products.

According to known art, no publication or patent disclosed the plantaricin A type (PlnA) synthesis as a response mechanism to co-culture of two lactic acid bacteria (for example. *L. plantarum* and *L*. *sanfranciscensis*) occurring in the same alimentary ecosystem, as "natural sourdough" used for the production of baked leavened products. Further according to literature, it has not been reported an increment of plantaricin A synthesis in co-culture (inter-species) with respect to mono-culture conditions (intra-species). In addition, as above reported, according to known processes very expensive and complex culture media are used.

Moreover, it has been surprisingly discovered that the biomass obtained according to the process of the invention comprising one or more plantaricins in association with *L. plantarum* DC400 (DSM 23213) and *L*. *rossiae* DPPMA174 (DSM 23214) lactic acid bacteria exerts an higher effectiveness than plantaricin alone in enhancing the barrier function at level of epidermis or intestinal wall.

The lactic acid bacteria according to the present invention belong to the *Lactobacillus* species and have been isolated from "natural sourdoughs" for typical bread production in South Italy.

A biotechnological protocol involving the co-culture of said two bacteria in CDM (Chemically Defined Medium), WFH (Wheat Flour Hydrolyzate) (Gobbetti, 1998. The sourdough microflora: interactions of lactic acid bacteria and sourdoughs. Trends Food Sci Technol 9:267-274), grape must (diluted at 1% of soluble carbohydrates, added with 0.5% of maltose and 0.5% of sourdough extract, pH 5.6), milk serum (added with 0.5% of maltose and 0.5% of sourdough extract, pH 5.6) or aqueous extracts of vegetable and fruit products (added with 0.5% of maltose and 0.5% of sourdough extract, pH 5.6) for 18 - 24 hours at 30 - 37°C has been standardized and optimized. At the end of the culture, the cells can be removed or not from the broth-culture by centrifugation, then the supernatant is subjected to a dehydrating process by drying or freeze-drying.

A scheme of the biotechnological protocol for the formulation of the preparation based on plantaricin A is described below.

| |
|---|
| Propagation of lactic acid bacteria cultures at 30°C for 24 hours, washing, suspension in water at 9.0 log ufc/ml cell density and co-inoculation (1-4%) of the culture medium (CDM, WFH, grape must, milk serum, aqueous extracts of fruit and vegetable products) |
| ↓ |
| Culture at 30 - 37°C for 18 - 24 hours |
| ↓ |
| Removal of the cells by centrifugation |
| ↓ |
| Dehydration of the supernatant by drying or freeze-drying |
| ↓ |
| Formulation of the preparation for skin care product application |

When a culture of *L. plantarum* DC400 (DSM 23213) and *L*. *rossiae* DPPMA174 (DSM 23214) under co-culture conditions on any of above said substrates is carried out the synthesis of plantaricin A at concentration from 2.5 to 4.0 µg/mL has been detected. Under mono-culture conditions, the concentration of plantaricin A synthesized by *L. plantarum* DC400 (DSM 23213) is about 0.06 µg/mL. Under co-culture conditions with other lactic acid bacteria species (for example *Pediococcus pentosaceus, Lactobacillus pentosus, Lactobacillus brevis, Lactobacillus rossiae*, *Lactobacillus rhamnosus*) the synthesis of plantaricin A is remarkably lower. Under co-culture conditions with *L*. *rossiae* DPPMA174 (DSM 23214) the synthesis of other peptide pheromones, as plantaricin type K and N has been detected, although at lower concentrations than plantaricin A, and particularly in the range from 0.02 to 0.06 µg/ml. According to one possible formulation, the application of 2.5 µg/ml of plantaricin A stimulated the barrier functions as proved using a reconstructed epidermis model (SkinEthic®) and Transepitheliall Electric Resistance (TEER) assay. Similar results have been obtained using Caco-2/TC7 intestinal cells suitable to reproduce the intestinal mucosa

According to complementary analyses carried out using microbiological, chromatographic techniques and in vitro and ex-vivo assays on cell cultures, the co-culture of *L. plantarum* DC400 (DSM 23213) and *L. rossiae* DPPMA174 (DSM 23214), never used in prior studies, according to the present invention, allows: (i) the synthesis of signal molecules involved in inter-species cell communication mechanisms at a concentration not detectable in the presence of other lactic acid bacteria associations within a single ecosystem; (ii) the synthesis of plantaricin A, and other plantaricins (K and N), also using low cost substrates; and (iii) a protective effect enhancing the barrier function at epidermis and intestinal cell level, thus demonstrating that signal molecules synthesized by prokaryotic cells are detected also by eukaryotic cells.

It is therefore a specific object of the present invention a biotechnological process for the synthesis of a biomass comprising or consisting of at least one plantaricin selected from plantaricins A, K or N, preferably A, or mixtures thereof and *Lactobacillus plantarum* DSM 23213 and *Lactobacillus rossiae* DSM 23214 lactic acid bacteria or for the preparation of one or more plantaricins selected from plantaricins A, K or N, preferably A, or mixtures thereof, said process comprising or consisting of the following steps:
a) culture propagation of *Lactobacillus plantarum* DSM 23213 and *Lactobacillus rossiae* DSM 23214 lactic acid bacteria;
b) co-inoculation of a substrate selected from the group consisting of Chemically Defined Medium (CDM), Wheat Flour Hydrolyzate (WFH), grape must, milk serum or fruit and vegetable product extracts, with aqueous suspension of lactic acid bacteria as defined in step a);
c) incubation; and, optionally,
d) centrifugation of the culture broth in order to remove the lactic acid bacteria cells.

Particularly, the cell density of suspension from step a) can be about 9,0 Log ufc/ml for each lactic acid bacteria species and it is added to the substrate at percentage ranging from 1 to 4% based on the substrate volume. The incubation step can be carried out at a temperature from 30 to 37°C, preferably 30°C, for 18 - 24 hours, preferably 18 hours, while the centrifugation can be carried out at 10000 x g for 15 min at 4°C.

The process according to this invention can further comprise a step e) for dehydration of the supernatant obtained from step d) by drying or freeze-drying.

It is a further object of the present invention a biomass obtainable or obtained according to above said process, comprising or consisting of at least one plantaricin selected from plantaricins A, K or N, preferably A, or mixtures thereof and *Lactobacillus plantarum* DSM 23213 e *Lactobacillus rossiae* DSM 23214 lactic acid bacteria.

The present invention further concerns a pharmaceutical or cosmetic composition comprising or consisting of the biomass as above defined, as an active principle, in association with one or more pharmaceutically acceptable excipients and/or adjuvants.

A particular aspect of the present invention further refers to the use of the biomass or composition as above defined for the preparation of a medicament for the treatment or the prevention of the barrier function damage of epidermis or intestinal wall or for wound healing.

*Lactobacillus plantarum* DSM 23213 or *Lactobacillus rossiae* DSM 23214 lactic acid bacteria or mixtures thereof are a further object of the present invention.

Further the present invention refers to the use of one or more plantaricins selected from plantaricins A, K or N, preferably A, for the preparation of a medicament for the treatment or the prevention of the barrier function damage of epidermis or intestinal wall or for wound healing.

The present invention now will be described by an illustrative but not a limitative way, according to preferred embodiments thereof, particularly with reference to the enclosed drawings.

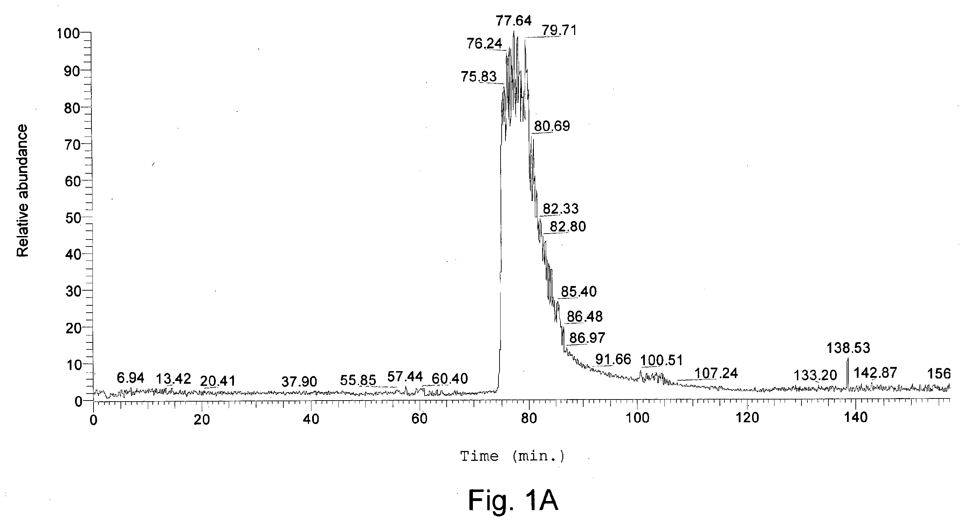

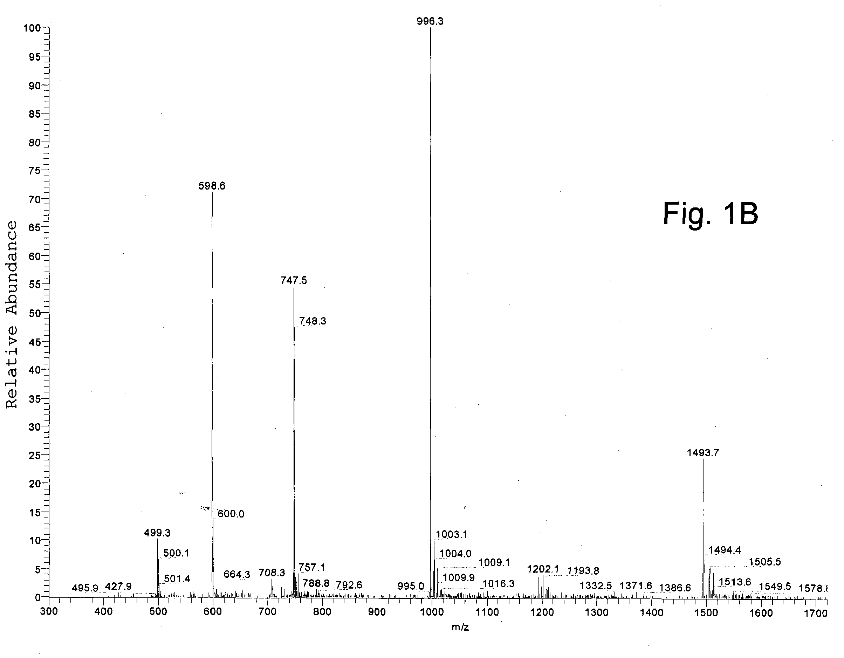

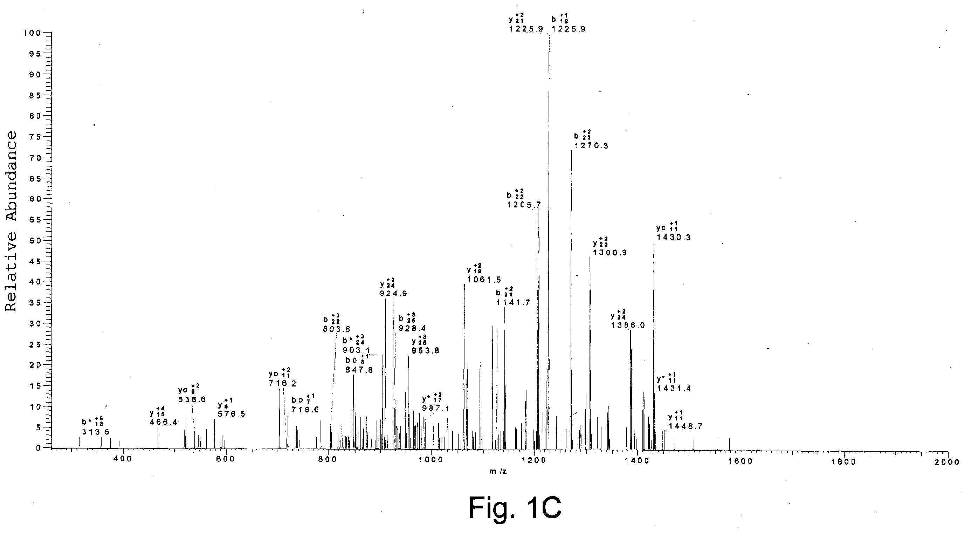

Figure 1 a shows results of electrospray-ionization (ESI) ion trap MS (nano-ESI/MS-MS) coupled Multidimensional HPLC (MDLC) analysis of free cell supernatant from broth-culture obtained by co-culture of *Lactobacillus plantarum* DC400 (DSM 23213) and *Lactobacillus rossiae* DPPMA174 (DSM 23214). Figure 1b shows the chromatogram obtained from real chromatogram of Figure 1a based on the specific acquisition time with m/z ratios relative to the plantaricin A. Figure 1c shows MS/MS spectrum detecting species based on 1493.7 m/z ratio as observed in chromatogram of Figure 1 a.

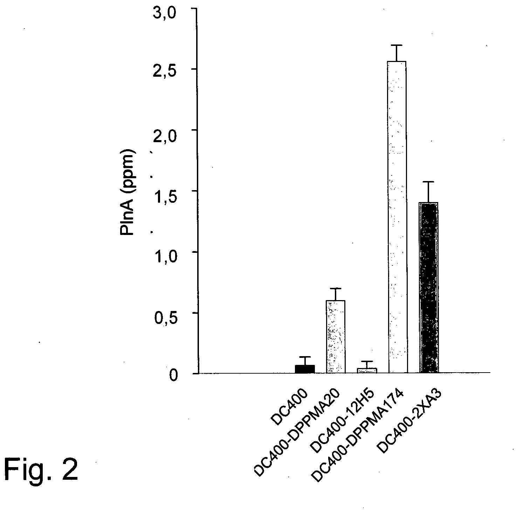

Figure 2 shows concentration of plantaricin A synthesized from *Lactobacillus plantarum* DC400 (DC400), *L. plantarum* DPPMA 20 (DPPMA20), *Lactobacillus pentosus* 12H5 (12H5), *Lactobacillus rossiae* DPPMA174 (DPPMA174) and *Pediococcus pentosaceus* 2XA£ (2XA3) mono-cultures and *L. plantarum* DC400 with *L. plantarum* DPPMA20 (DC400-DPPMA20), *L. pentosus* 12H5 (DC400-12H5); *L. rossiae* DPPMA174 (DC400-DPPMA174) or *P. pentosaceus* 2XA3 (DC400-2XA3) co-cultures. Data is the average of three triplicate experiments.

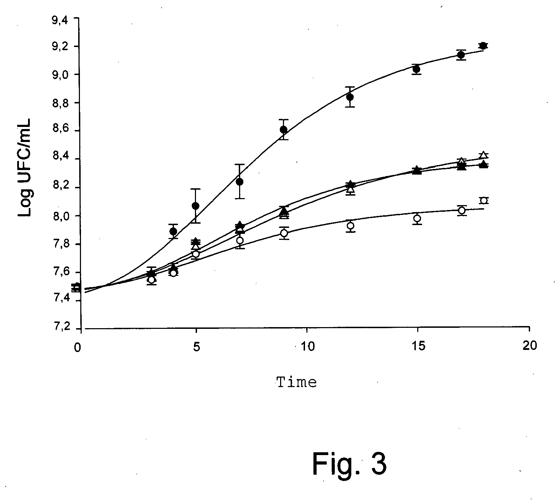

Figure 3 shows growth kinetics of *Lactobacillus rossiae* DPPMA174. Mono-culture (●); co-culture with *Lactobacillus plantarum* DC400 (○); mono-culture with purified plantaricin A (2.5 µg/ml) (Δ); and mono-culture with chemically synthesized plantaricin A (2.5 µg/ml) (▲). Purified plantaricin A corresponds to that synthesized by co-culture of *L*. *plantarum* DC400 and *L. rossiae* DPPMA174. Data is the average of three triplicate experiments.

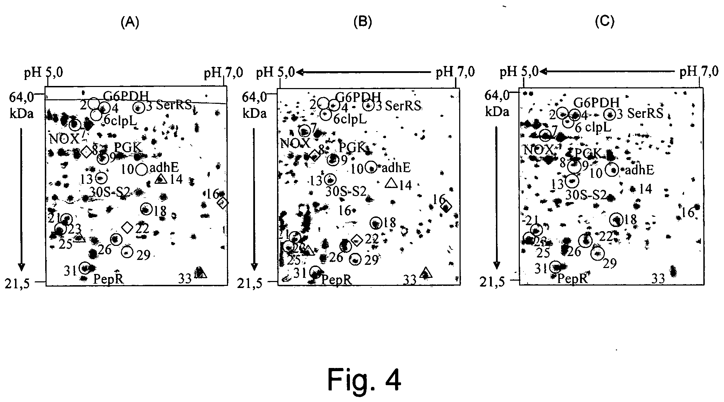

Figure 4 shows portions of gel relating to electrophoretic two-dimensional analysis of *Lactobacillus rossiae* DPPMA174 expressed proteins after 18 hour culture. Panel A, mono-culture; panel B, co-culture with *Lactobacillus plantarum* DC400; and panel C, mono-culture in the presence of purified plantaricin A (2.5 µg/ml). Oval or triangle marked numbers refer to proteins displaying an expression level increase or decrease in the culture with *L. plantarum* DC400 or purified plantaricin A. Rhomb or double triangle marked numbers refer to proteins displaying an expression level increase or decrease only in the co-culture with *L*. *plantarum* DC400.

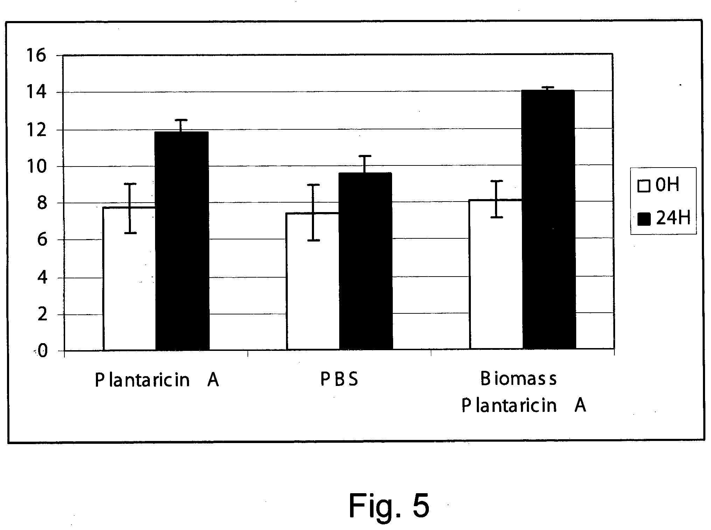

Figure 5 shows Transepithelial Electric Resistance (TEER) (Ohms x cm²) of reconstructed epidermis (SkinEthic®) after exposure for 0 and 24 hours to PBS buffer, plantaricin A (2.5 µg/ml) or biomass containing 2.5 µg/ml of plantaricin A, respectively. Data is the average of three triplicate experiments.

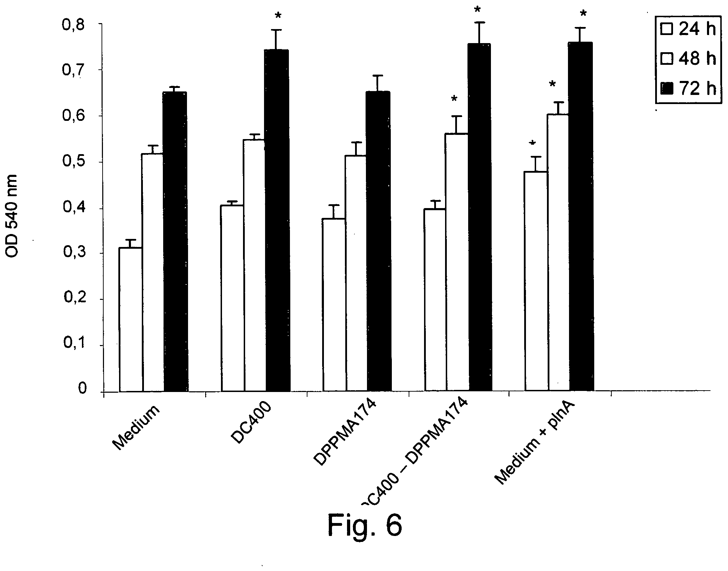

Figure 6 shows Caco2/TC7 cell viability measured as Neutral Red absorption after 24, 48 and 72 hours of incubation with purified plantaricin A (2.5 µg/ml) produced by *Lactobacillus plantarum* DC400 (DC400) mono-culture or with *Lactobacillus rossiae* DPPMA174 (DC400-DPPMA174) co-culture. The purified fraction of *L. rossiae* DPPMA174 mono-culture eluted according to chromatographic conditions as for plantaricin A, has been used as negative control (DPPMA174). Another negative control is DMEM culture medium (DMEM). Chemically synthesized plantaricin A (2.5 µg/ml) has been used as positive control (PlnA). Data is the average of three triplicate experiments. Asterisk indicates significant differences (*P<0.01*) with respect to negative control.

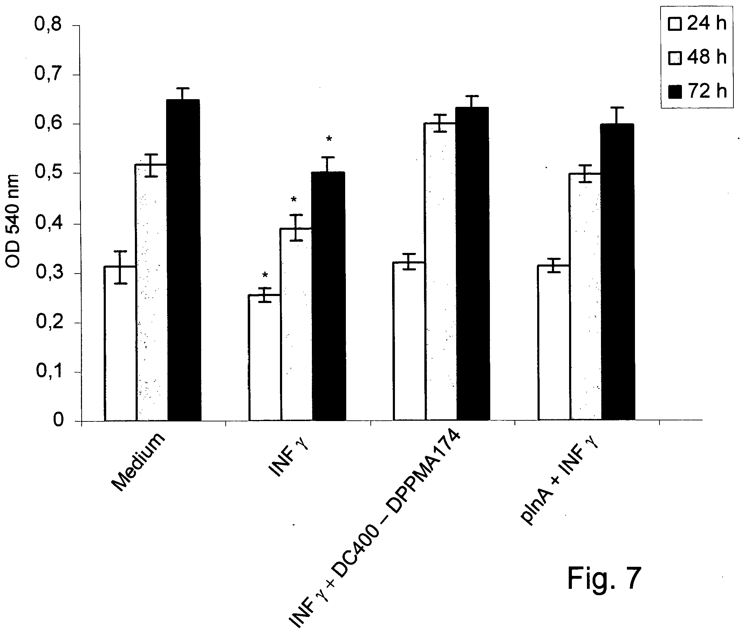

Figure 7 shows Caco2/TC7 cell viability measured as absorption of Neutral Red after 24, 48 and 72 hours of incubation with γ-interpheron (IFN-γ) (1000 U/ml) alone and with IFN-γ + purified plantaricin A (2.5 µg/ml) (IFN-γ + DC400-DPPMA174). Purified plantaricin is from *Lactobacillus plantarum* DC400 and *Lactobacillus rossiae* DPPMA174 co-culture. DMEM medium culture has been used as negative control (DMEM). Chemically synthesized plantaricin A (2.5 µg/ml) together with IFN-γ has been used as positive control (PlnA). Data is the average of three triplicate experiments. Asterisk indicates significant differences (*P<0.01*) with respect to negative control.

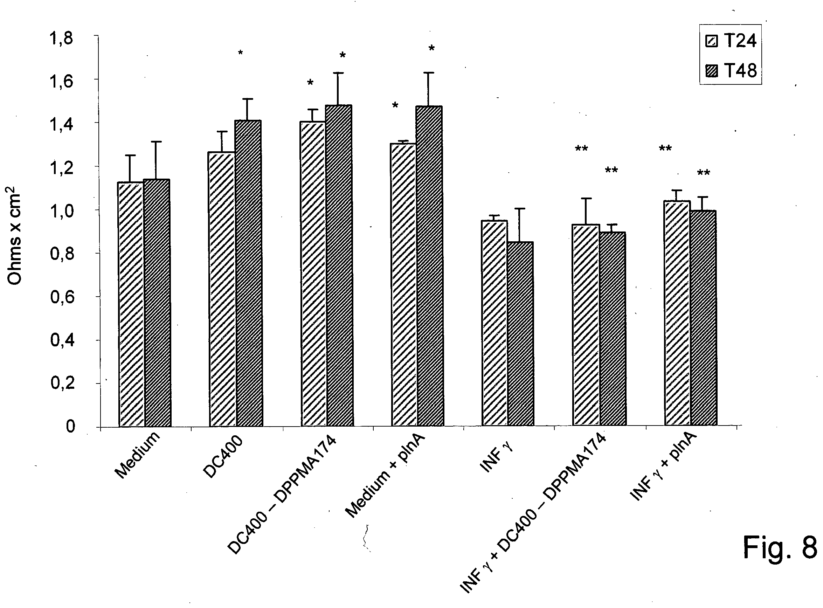

Figure 8 shows Transepithelial Electric Resistance (TEER) (Ohms x cm²) of Caco2/TC7 cells after 24 and 48 hours of incubation. The incubation has been carried out with: purified plantaricin A (2.5 µg/ml) from *Lactobacillus plantarum* DC400 (DC400) mono-culture; purified plantaricin A (2.5 µg/ml) from *L. plantarum* DC400 and *Lactobacillus rossiae* DPPMA174 (DC400-DPPMA174) co-culture; chemically synthesized plantaricin A (2.5 µg/ml) (PlnA); γ-interpheron (IFN-γ) (1000 U/ml) and purified plantaricin A from *L. plantarum* DC400 and *L. rossiae* DPPMA174 (IFN-γ + DC400-DPPMA174) co-culture; and IFN-γ and chemically synthesized plantaricin A (PlnA + IFN-γ). DMEM culture medium (DMEM) has been used as negative control. Data is the average of three triplicate experiments. Asterisk indicates significant differences (*P<0.01*) with respect to negative control.

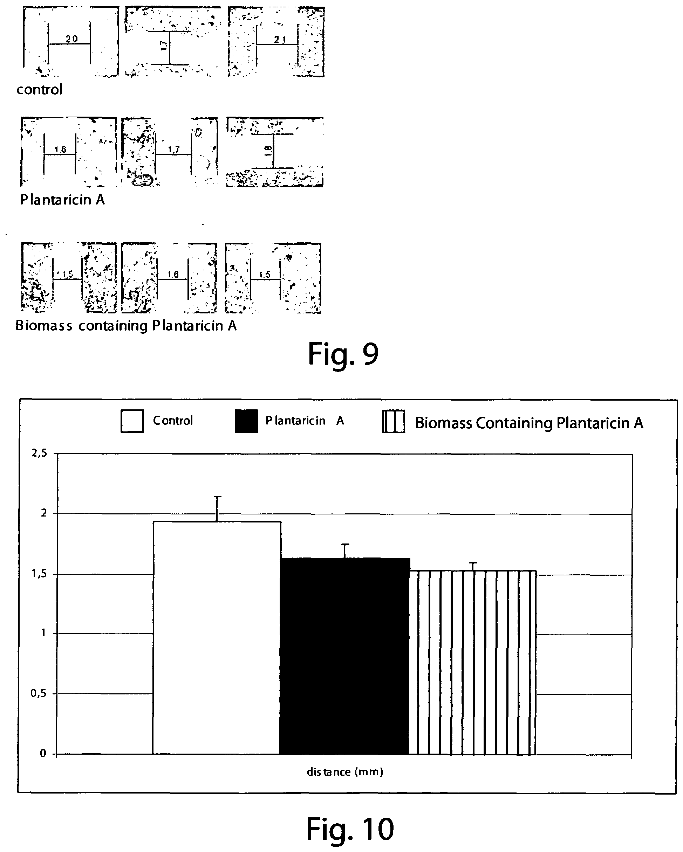

Figure 9 shows results of wound healing assays on keratinocytes treated with the control, plantaricin A or plantaricin A containing biomass, respectively.
Figure 10 shows the distance between the margins of a wound treated with control, plantaricin A or plantaricin A containing biomass.

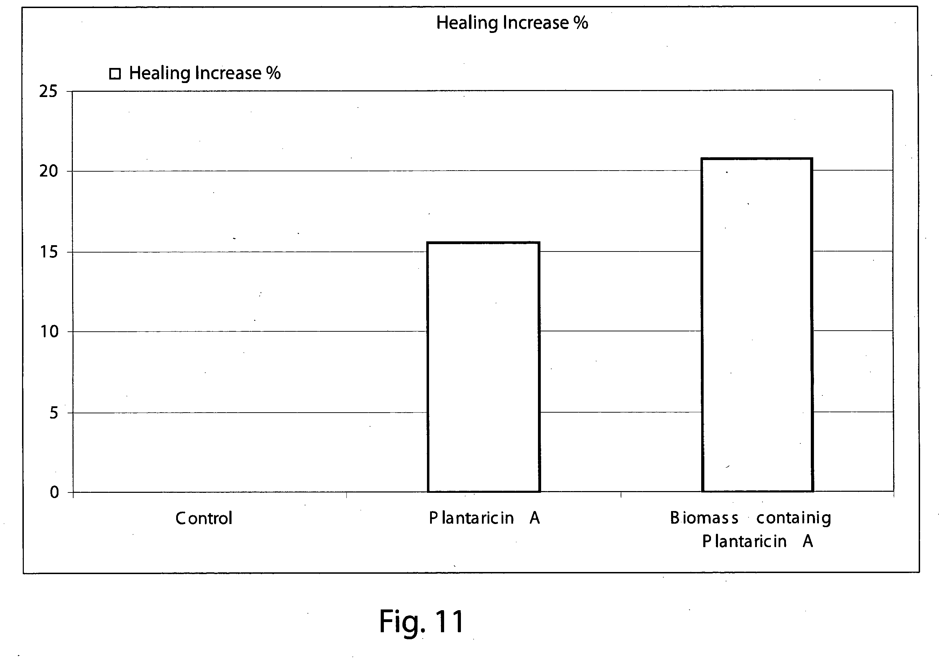

Figure 11 shows increment percentage of healing for the wound treated with the control, plantaricin A or plantaricin A containing biomass.

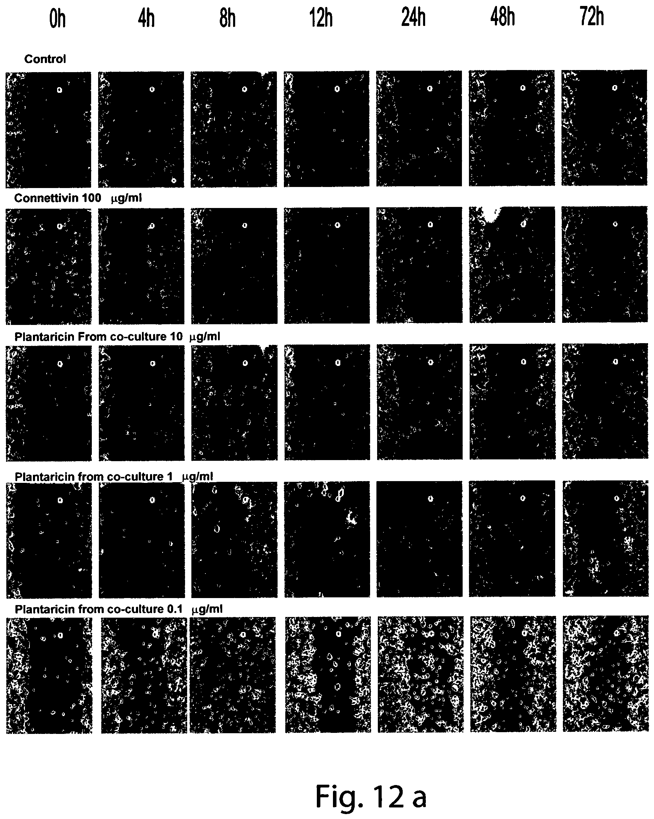

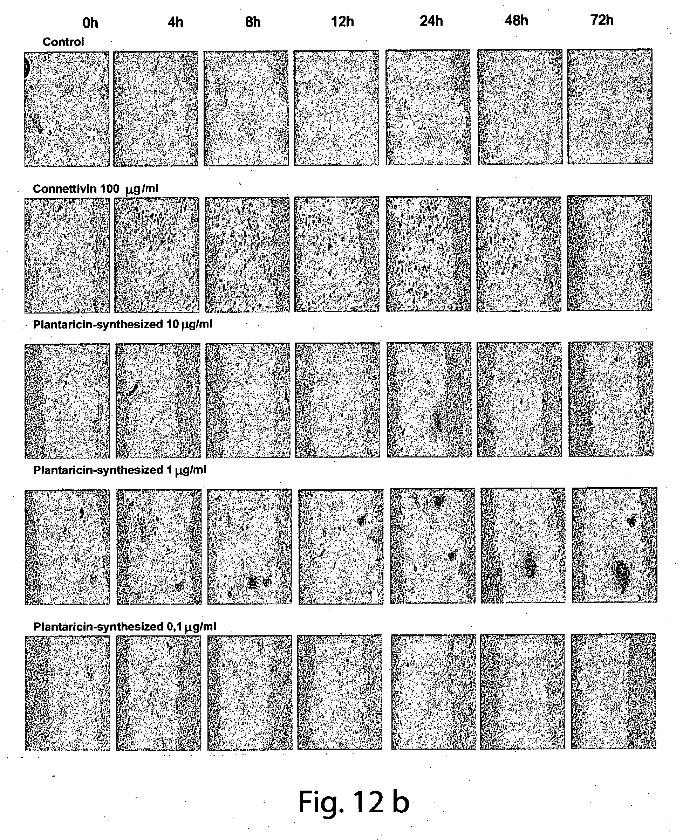

Figure 12 shows representative images of human 2544 NCTC keratinocyte monolayer treated, following the cut, with co-cultured Plantaricin (a) and synthetic Plantaricin (b), at considered different time intervals. The monolayer cut has been carried out using 200 µl pipette tip. The cells have been treated with co-cultured and synthetic Plantaricin independently and at concentrations of 0.1, 1 and 10 µg/ml.

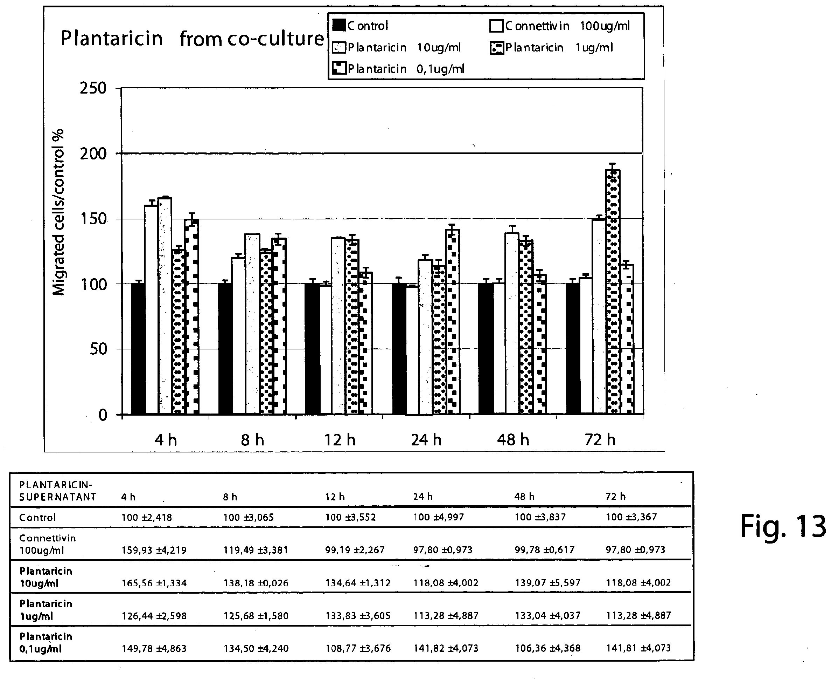

Figure 13 shows the percentage of cells migrated through the cut area after treatment with co-cultured Plantaricin (0.1, 1 and 10 µg/ml). The migration percentages have been determined measuring the clear area between the two cut lines at 0, 4, 8, 12, 24, 48 and 72 hours after the cut. Bars represent average ± S.E.M of two triplicate independent experiments.

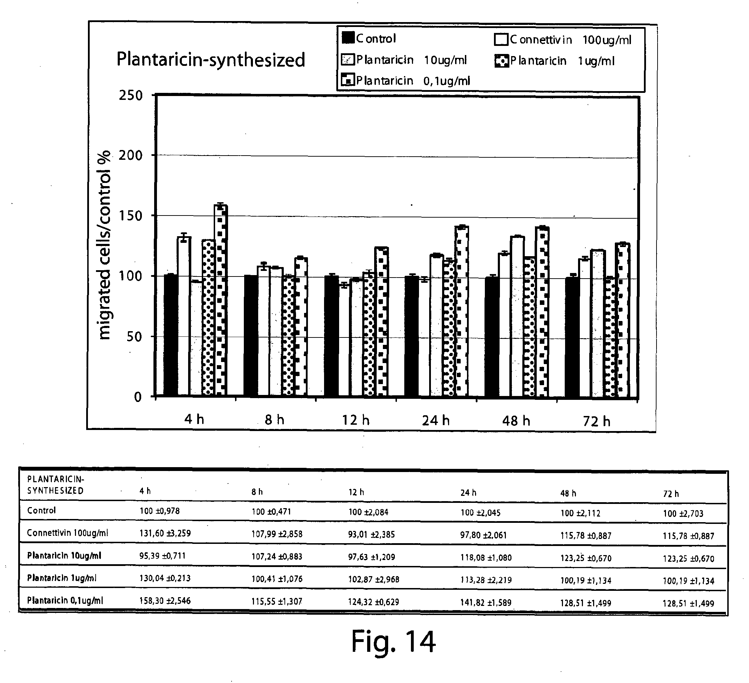

Figure 14 shows the percentage of cells migrated through the cut area at starting time after treatment with synthetic Plantaricin (0.1, 1 and 10 µg/ml). The migration percentages have been determined measuring the clear area between the two cut lines at 0, 4, 8, 12, 24, 48 and 72 hours after the cut compared to initial clear area. Bars represent average ± S.E.M of two triplicate independent experiments.

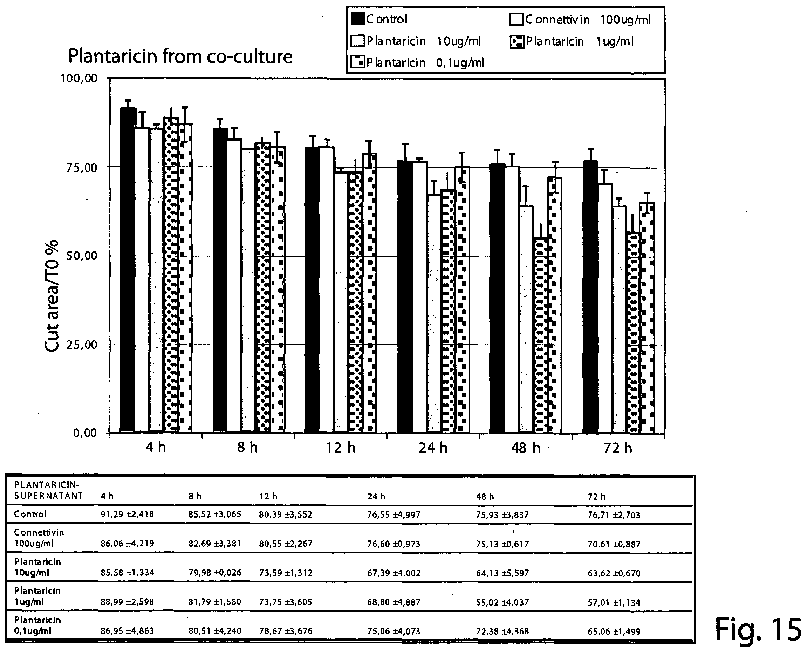

Figure 15 shows the percentage of clear area with respect to initial cut area after treatment of human NCTC2544 keratinocyte monolayer with co-cultured Plantaricin (0.1, 1 and 10 µg/ml). The migration percentages have been determined measuring the clear area between the two cut lines at 0, 4, 8, 12, 24, 48 and 72 hours after the cut compared to initial clear area. Bars represent average ± S.E.M of two triplicate independent experiments.

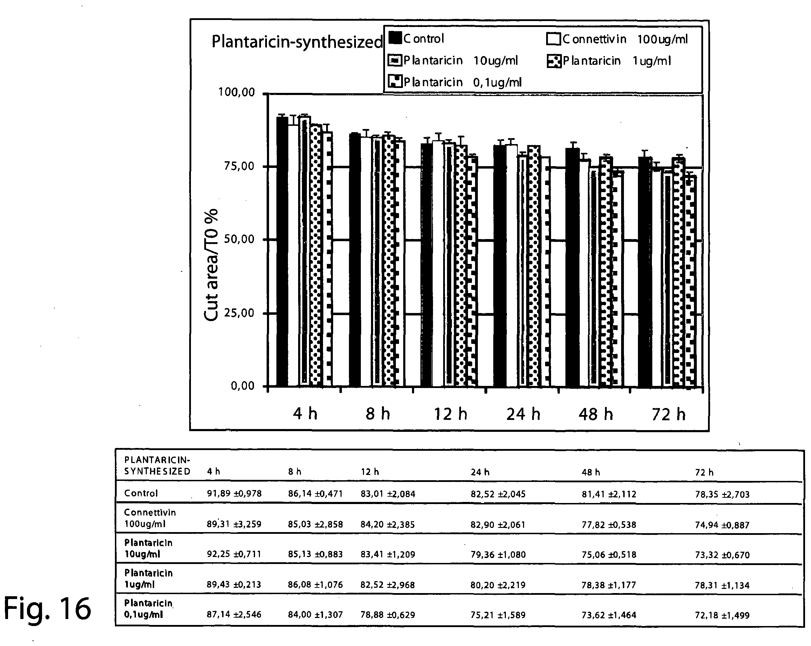

Figure 16 shows the percentage of clear area with respect to the initial cut area after treatment of human NCTC2544 keratinocyte monolayer with synthetic Plantaricin (0.1, 1 and 10 µg/ml). The percentages have been determined measuring the clear area between the two cut lines at 0, 4, 8, 12, 24, 48 and 72 hours after the cut compared to initial clear area. Bars represent average ± S.E.M of two triplicate independent experiments.

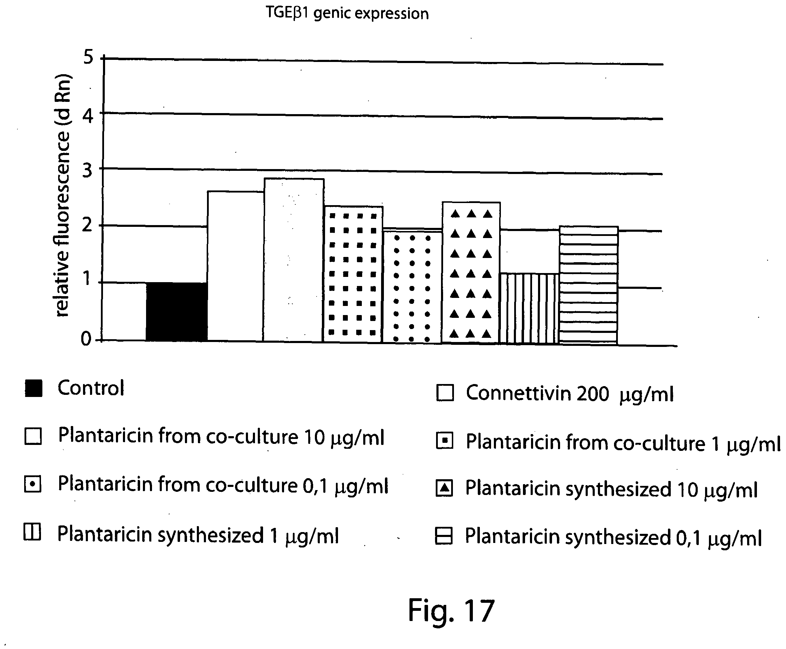

Figure 17 shows the effect of co-cultured Plantaricin (0,1, 1 and 10 µg/ml), synthetic Plantaricin (0.1, 1 and 10 µg/ml) and Connectivine (200 µg/ml) on TGFβ1□ expression in human NCTC 2544 keratinocyte monolayer, incised in order to have a 200 µl pipette tip cut.

### Example 1: Synthesis and purification of plantaricin type peptide pheromones and study of the effects thereof on epidermis and Caco-2 cells.

*L. plantarum* DC400 (DSM 23213) and *L. rossiae* DPPMA174 (DSM) from the Collezione di Colture del Dipartimento di Protezione delle Piante e Microbiologia Applicata dell'Università degli Studi di Bari, previously isolated from "natural sourdoughs", have been propagated at 30°C for 24 hours in modified MRS media (mMRS), containing, in addition to usual ingredients, 5% maltose and 10% sourdough water - final pH 5.6.

24 h cultured cells, collected by centrifugation (10.000 x *g* for 15 min at 4°C), washed twice with 50 mM phosphate buffer, pH 7.0 and resuspended in water at cell density of 9.0 log ufc/ml have been inoculated (4%, for each species) in mono- or co-culture conditions on WFH culture media (Gobbetti, 1998. The sourdough microflora: interactions of lactic acid bacteria and sourdoughs. Trends Food Ski Technol 9:267 - 274). Same procedure has been applied and same results, successively described, have been obtained using grape must (diluted at 1% of soluble carbohydrates, added with 0.5% maltose and 0.5% sourdough extract, pH 5.6), milk serum (see previous integrations) or aqueous extracts of vegetable and fruit products (see previous integrations) as culture substrates. The incubation is carried out for 18 hours at 30°C. After cell removal by centrifugation (10.000 x g for 10 min at 4°C), mono- and co-culture supernatants have been added with trifluoroacetic acid (0.05%) and centrifuged at 10.000 x g for 10 min. The supernatant has been filtered using 0.22 µm pore filters. HPLC analysis has been carried out using AKTA Purifier (GE Healthcare) apparatus equipped with a detector operating at 214 nm and using a reverse phase C18 XTerra column (Waters, Mildford). Mixture of water, 2-propanol and trifluoroacetic acid (0.05%) has been used as mobile phase. All type A plantaricin containing fractions have been analyzed using ESI-ion trap MS mass spectrometer coupled multidimensional chromatograph (MDLC). The analysis conditions for the identification and quantification of plantaricins A, K and N are according to Di Cagno et al. (Di Cagno et al., 2010. Quorum sensing in sourdough Lactobacillus plantarum DC400 (DSM 23213): induction of plantaricin A (PlnA) under co-cultivation with other lactic acid bacteria and effect of PlnA on bacterial and Caco-2 cells. Proteomics in press).

### (2) Growth kinetics of Lactobacillus rossiae DPPMA174 (DSM 23214)

Growth kinetic data of *L. rossiae* DPPMA174 (DSM 23214) have been processed using Gompertz equation, successively modified (Zwietering et al., 1990. Modelling of bacterial growth curve. Appl Environ Microbiol 56: 1875-1881). Cell counting has been carried out by plating on SDB culture medium at 30°C for 48 hours. Cell viability and number of damaged and/or died cells have been determined by means of LIVE/DEAD bacLight Bacterial Viability kit (Molecular Probes, INc., Cambridge).

### (3) Two-dimensional electrophoretic analysis and proteins identification

Two-dimensional electrophoretic analysis of cytoplasmic proteins from mono- or co-cultured *L. rossiae* DPPMA174 (DSM 23214) has been carried out using immobiline-polyacrylamide system (De Angelis et al., 2005. Biochim. Biophys. Acta. 1762:80-93). Four gels for each condition have been analysed and the data have been standardized according to procedure by Bini et al. (Bini et al., 1997. Protein expression profiles in human breast ductal carcinoma and histologically normal tissue. Electrophoresis. 18:2831-2841).

Protein identification has been carried out using LC-ESI-MS/MS analysis and comparison of obtained sequences to various databases (National Center for Biotechnology Information, Bethesda, MD, USA; ProFound, http://www.prowl.rockefeller.edu/cgibin/ProFound).

### (4) Assays on reconstructed epidermis and TEER (Transepithelial Electric Resistance) determination

Reconstructed human epidermis SkinEthic® (Reconstructed Human Epidermis) consists of normal kenatinocytes from multi-layer human epidermis. It is a completely differentiated epidermis from human keratinocytes culture in a chemically defined medium (MCDM 153), without calf serum addition, on an inert porous polycarbonate support at air-liquid interface over 17 days. At this growth step the morphologic analysis shows a multi-layered viable epidermis and corneous layer consisting of more than ten compact cellular layers. Reconstructed human epidermis SkinEthic® has been used according to previously described protocol (Di Cagno et al., 2009. Synthesis of γ-amino butyric acid (GABA) by Lactobacillus plantarum DSMZ19463: functional grape must beverage and dermatological application. Appl Biotechnol Microbiol DOI: 10.1007/s00253-009-23704)..

TEER determination has been carried out using Millicell-ERS Volthommeter (Di Cagno et al., 2010. Quorum sensing in sourdough Lactobacillus plantarum DC400: induction of plantaricin A (PlnA) under co-cultivation with other lactic acid bacteria and effect of PlnA on bacterial and Caco-2 cells. Proteomics in press).

### (5) Assays on Caco-2/TC7 cells

Human Caco-2/TC7 cells (TC7 clone) have been cultured in Dulbecco medium (DMEM), added with calf serum (10%), not essential amino acids (1%), gentamycin/streptomycin (50 µg/ml), glutamine (2 mM) and 4-2-hydroxyethyl-1-piperazinil-etansulfonic acid (1%) (Di Cagno et al., 2010. Quorum sensing in sourdough Lactobacillus plantarum DC400: induction of plantaricin A (PlnA) under co-cultivation with other lactic acid bacteria and effect of PlnA on bacterial and Caco-2 cells. Proteomics in press). Cell viability has been determined with absorption assay using Neutral Red dye (Balls et al., 1987. Approaches to validation alternative methods in toxicology. In: Goldber A.M. (Ed). N.Y. Academic Press pp. 45-58). After 24 - 72 hour treatment with various preparations, cells have been washed with PBS buffer and incubated for 4 hours at 37°C with a Neutral Red solution (33 mg/l). Successively, the cells have been again washed with PBS buffer and treated with lysis solution (50% ethanol in 1% acetic acid containing water). Plate reading has been carried out using Novapath plate reader (Biorad, Hercules, CA). Di Cagno et al., 2010. Quorum sensing in sourdough Lactobacillus plantarum DC400: induction of plantaricin A (PlnA) under co-cultivation conditions with other lactic acid bacteria and effect of PlnA on bacterial and Caco-2 cells. Proteomics in press).

For TEER determination Caco-2/TC7 cells have been inoculated (7.5 x 10⁴ cells/ml) in a 24 well plate and a polyethylene filter (0.4 µm pore). Before the treatment, the cells have been incubated for 21 days at 37°C. Treatments with various preparations have been carried out for 18, 24 and 48 hours. Integrity of the cellular layer therefore has been determined by means of TEER determination.

### Results

### (1) Synthesis and purification of plantaricin type peptide pheromones

After 18 hour growth in WFH medium culture the cell density of *L*. *plantarum* DC400 (DSM 23213) mono-culture was 9.27 ± 0.18 log ufc/ml. µₘₐₓ and λ values were, respectively, about 0.27 log ufc/ml/h and 3.77 h. The cell density of lactic acid bacteria changed from 9.0 ± 0.05 (*L. brevis* CR13) to 9.43 ± 0.31 log ufc/ml (*L. plantarum* DPPMA20). µₘₐₓ values varied from 0.11 ± 0.05 (*L. pentosus* 12H5) to 0.15 ± 0.04 log ufc/ml/h (*L*. *plantarum* DPPMA20), as weel as λ value changed from 0.37 ± 0.07 (P. *pentosaceus* 2XA3) to 4.20 ± 0.36 h (*L. rossiae* DPPMA174 (DSM 23214)). In comparison to mono-culture, the cell density of *L. plantarum* DC400 (DSM 23213) did non change significantly (*P>0.05*) (9.06 ± 0.34 - 9.28 ± 0.42 log ufc/ml) when the micro-organism has been co-cultured with the other lactic acid bacteria. Also cell yield for *L. plantarum* DPPMA20, *Lactobacillus paralimentarius* 8D, *L. pentosus* 12H5, *Lactobacillus reuteri* e *Weissella cibaria* 10XA16 has not been conditioned by the co-culture conditions. On the contrary, the cell density of *L. rossiae* DPPMA174 (DSM 23214) and *P. pentosaceus* 2XA3 is remarkably decreased (P<0.05) (about 8.08 and 8.39 log cfu/ml) compared to mono-culture conditions. Generally µₘₐₓ values are decreased for all the lactic acid bacteria when co-cultured with *L. plantarum* DC400 (DSM 23213). With the exception of *L. plantarum* DPPMA20, also the λ latency phase is increased for all the lactic acid bacteria. Lactic acid bacteria strains that have shown an inhibition (*L. rossiae* DPPMA174 (DSM 23214) and *P*. *pentosaceus* 2XA) as a result *L. plantarum* DC400 (DSM 23213) co-culture and some strains not affected by co-culture conditions (*L*. *plantarum* DPPMA20 and *L. pentosus* 12H5) have been used in successive experiments.

In agreement with the previous results, the number of *L. rossiae* DPPMA174 (DSM 23214) viable cells is decreased from 9.0 ± 0.28 to 8.32 ± 0.25 log cellule/ml from mono-culture to co-culture conditions with *L*. *plantarum* DC400 (DSM 23213). Number of viable and culturable cells did not show significant differences (*P>0.05*). Again with reference to mono-culture conditions, the number of dead or damaged *L. rossiae* DPPMA174 (DSM 23214) cells is significantly (*P<0.05*) increased in co-culture conditions with *L. plantarum* DC400 (DSM 23213). Also the number of culturable *P. pentosaceus* cells is significantly (*P<0.05*) decreased when the lactic bacterium has been co-cultured with *L. plantarum* DC400 (DSM 23213).

After cell removal, the mono- and co-culture supernatants are used for the determination of plantaricin type peptide pheromones using nano-ESI/MS-MS mass spectrometry coupled MDLC analysis. Figure 1a shows full-scan chromatogram of *L. plantarum* DC400 (DSM 23213) and *L. rossiae* DPPMA174 (DSM 23214) co-culture sample. Because of matrix complexity, it has been possible to identify some species, while on the contrary it was difficult to carry out a complete separation of adjacent peaks. However, it was possible to separate the co-eluted species by signal filtration in correspondence of particular m/z values. An example of identified species is reported in Figure 1 b. MS/MS spectra have been obtained for each species. For example Figure 1c shows the spectrum corresponding to 1493.7 m/z value, selected for sample from L. *plantarum* DC400 (DSM 23213) and *L. rossiae* DPPMA174 (DSM 23214) co-culture. For the sequence search on NCBlnr database the following parameters have been specified: genus (*Lactobacillus*), m/z tolerance ratio for ion recognition (0.2 Da) and instrumentation used for analysis. The presence of plantaricin A (SEQ ID NO:1 Lys-Ser-Ser-Ala-Tyr-Ser-Leu-Gln-Met-Gly-Ala-Thr-Ala-Ile-Lys-Gln-Val-Lys-Lys-Leu-Phe-Lys-Lys-Trp-Gly-Trp) has been observed both for *L. plantarum* DC400 (DSM 23213) and DPPMA20 mono-cultures and all co-cultures wherein DC400 strain has been cultured with other lactic acid bacteria.

Based on previous results the samples from mono- and co-cultures have been purified using 4 chromatographic runs and further analyzed using nano-ESI-MS in order to exclude other peptide contamination. The concentration of *L. plantarum* DC400 (DSM 23213) synthesized plantaricin A has been determined by chromatographic analysis, using a reverse phase C18 XTerra column (Waters, Mildford). and OPA method. Figure 2 shows the concentration of plantaricin A under various conditions. It is possible to observe that the synthesis yield of the peptide pheromone is increased from mono-culture (about 0.06 µg/ml) to co-culture conditions in the presence of *L. rossiae* DPPMA174 (DSM 23214) (about 2.5 µg/ml). Under certain experimental conditions the concentration of plantaricin A is about 4.0 µg/ml. Although under co-culture conditions with other lactic acid bacteria species the production of plantaricin A has been observed, the obtained amount is remarkably lower than for *L. plantarum* DC400 (DSM 23213) and *L. rossiae* DPPMA174 (DSM 23214) co-culture.

This result proves that the synthesis of the peptide pheromone is specific in the presence of specific microbial interactions suitable to induce the release of signal molecules. The synthesis of plantaricin A starts during the intermediate exponential growth phase (about 7 hours) and increases up to the end of the exponential phase (about 12 hours). Although at lower concentrations, i.e. about 0.02-0.06 µg/ml, also type K and N plantaricins have been detected only in *L. plantarum* DC400 (DSM 23213) and *L. rossiae* DPPMA174 (DSM 23214) co-culture. Same results have been obtained using CDM, grape must, milk serum or fruit and vegetable product aqueous extracts as substrate for the co-culture cultivation.

### (2) Growth kinetics of Lactobacillus rossiae DPPMA174 (DSM 23214)

*L. rossiae* DPPMA174 (DSM 23214) has been cultivated on WFH culture medium added with 2.5 µg/ml of purified or chemically synthesized plantaricin A. In Figure 3 it is observed that the presence of purified plantaricin A resulted in a remarkable decrease of cell number, i.e. from 9.18 ± 0.26 (under mono-culture conditions) to 8.4 ± 0.14 log ufc/ml. Similar results have been obtained using chemically synthesized plantaricin A. In both these cases results similar to those found for *L*. *plantarum* DC400 (DSM 23213) and *L. rossiae* DPPMA174 (DSM 23214) co-culture have been observed. Damaged or died *L. rossiae* DPPMA174 (DSM 23214) cell number when culture has been carried out in the presence of purified or chemically synthesized plantaricin A has been significantly (*P<0.05*) higher than for mono-culture (about 8.80 ± 0.14 vs. 6.08 ± 0.22 log cells/ml).

Obtained data show that inhibitory effect of *L. plantarum* DC400 (DSM 23213) against *L. rossiae* DPPMA174 (DSM 23214) results from the synthesis of plantaricin A and, probably, other peptide pheromones belonging to the same chemical class.

### (3) Variation of the protein expression levels in L. rossiae DPPMA174 (DSM 23214)

In comparison to the mono-culture, the two-dimensional electrophoretic analysis of cytosol extracts of *L. rossiae* DPPMA174 (DSM 23214) cultivated in co-culture with *L. plantarum* DC400 (DSM 23213) or in the presence of purified plantaricin A has shown the variation of the expression level of 51 and 27 proteins, respectively. All the hyper-expressed proteins in the presence of the plantaricin A have been also hyper-expressed also under co-culture conditions. By way of example, Figure 4 shows portions of gels referring to mono-culture, co-culture with *L. plantarum* DC440 and mono-culture in the presence of purified plantaricin A conditions. Some of more hyper-expressed proteins have been identified using mass spectrometry analysis and found to be involved in protein biosynthesis (seryl-tRNA synthetase), energetic metabolism (glucose-6-phosphate dehydrogenase, phosphoglycerate mutase, acetaldehyde-CoA dehydrogenase, 6-phospho-gluconate dehydrogenase and β-phospho-gluco-mutase), katabolism of proteins and amino acids (ATP-dependent Clp proteinase and R aminopeptidase), environmental-stress responses (GroEL, GroES, S2 and S5 ribosomial proteins) and redox potential homeostasis (NADH oxidase). The majority of these proteins has been identified also in other lactic acid bacteria as a response to environmental stress conditions and/or cell communication mechanisms (Di Cagno et al., 2007. Cell-cell communication in sourdough lactic acid bacteria: a protomic study in Lactobacillus sanfranciscensis CB1. Proteomics 7:2430-2446). Particularly, glucose-6-phosphate dehydrogenase enzyme catalyzes the release of a fratricide pentapeptide in the mechanisms of Escherichia coli cell programmed death (Kolodkin-Gal et al., 2007. A linear pentapeptide is a quorum-sensing factor required for mazef-mediated cell death in Escherichia coli. Science 318:652-655).

The obtained results show that the inhibitory effect of *L. rossiae* DPPMA174 (DSM 23214) as a result of plantaricin A synthesis is based on cell communication mechanism and, probably, is suitable to trigger responses resulting in target microorganism death. These result prove a bactericidal valence of the signal molecule.

### (4) Assays on reconstructed epidermis and TEER (Transepithelial Electric Resistance) determination

Both a biomass sample from *Plantarum* DC400 (DSM 23213) and *L. rossiae* DPPMA174 (DSM 23214) co-culture with pantaricin A concentration of 2.5 µg/ml and co-culture deriving purified plantaricin A at same concentration of 2.5 µg/ml have been assayed for the treatment of reconstructed human epidermis according to SkinEthic® model. This model has been widely used and accepted by the scientific community (Di Cagno et al., 2009. Synthesis of γ-amino butyric acid (GABA) by Lactobacillus plantarum DSMZ19463: functional grape must beverage and dermatological application. Appl Biotechnol Microbiol DOI: 10.1007/s00253-009-23704). After 24 hour treatment TEER measurements have been carried out. This type of analysis, widely accepted by the international scientific community, evaluates the tissue corrosion taking as a reference the integrity of the corneous layer and barrier function. Particularly, using this evaluation it is possible to obtain information about the presence of a lamellar compact structure at corneous layer level, tight integral junctions and epidermal thickness. These factors as a whole define an efficient barrier function. Figure 5 shows that both in the presence of plantaricin containing biomass and subject co-culture deriving purified plantaricin A a significant increment (*P<0.05*) of TEER value occurs, thus demonstrating a protective activity of the molecule at cutaneous level. Same result has been obtained using chemically synthesized plantaricin A.

According to the current state of the art, this is the first example of application of a peptide pheromone, involved in bacteria cell communication mechanisms, suitable to be sensed by epidermis human cells resulting in stimulation of barrier function.

### (5) Assays on Caco-2/TC7 cells

Viability of Caco-2/TC7 cells has been evaluated as Neutral Red dye adsorption ability. In comparison to DMEM medium (negative control), the incubation for 24 - 72 hours with purified plantaricin A (2.5 µg/ml) remarkably increased the viability of Caco-2/TC7 cells (Figure 6). Same result has been obtained using chemically synthesized plantaricin A. No induction has been observed with treatment using sample deriving from *L*. *rossiae* DPPMA174 (DSM 23214) mono-culture purified fraction eluted according to same chromatographic conditions used for plantaricin A. As expected the exposure of Caco-2/TC7 cells to γ-interpheron (IFN-γ) resulted in a remarkable viability decrease (*P<0.05*) (Figure 7). On the contrary, the negative effect of IFN-γ is completely eliminated in the presence of a simultaneous treatment with purified or chemically synthesized plantaricin A. The addition of purified plantaricin A resulted in a remarkable increase (*P<0.05*) of TEER values for Caco-2/TC7 cells (Figure 8). The same result has been observed using Caco-2/TC7 cells. In comparison to DMEM medium, the addition of IFN-γ significantly (*P<0.05*) reduced TEER values. However, the addition of purified or chemically synthesized plantaricin A eliminated also in this case the negative effects.

Caco-2/TC7 cells are one of in vitro most used system in order to simulate the intestinal mucosa. Although neoplastic origin thereof, said cells are suitable to differentiate spontaneously in mature enterocytes and express brush border enzymes. Under culture conditions, Caco-2/TC7 cells are suitable to develop morphological and functional characteristics, including tight intercellular junctions, whose integrity is determined by TEER determinations (Sambuy et al., 2005. The Caco-2 cell line as a model of the intestinal barrier: influence of cell and culture-related factors on Cao-2 cell functional characteristics. Cell Biol Toxicol 21:1-26). The results of this study demonstrate that purified *L. plantarum* DC400 (DSM 23213) and *L. rossiae* DPPMA174 (DSM 23214) co-culture deriving or chemically synthesized plantaricin A is suitable to stimulate the barrier function of intestinal mucosa and prevent negative effects of γ-interpheron treatments.

### (6) Development of a biotechnological protocol for the synthesis of plantaricin A and use thereof in dermatological field

As above outlined in other part of the text, a biotechnological process for the synthesis of plantaricin A and use thereof in dermatological field has been developed. Said process comprises:
a) Cultivation of *L. plantarum* DC400 (DSM 23213) and *L. rossiae* DPPMA174 (DSM 23214) in pure culture on mMRS culture medium;
b) Cell collection, washing, re-suspension in WFH, CDM, grape must, milk serum or vegetable or fruit aqueous extracts suitably integrating for nutrient availability as culture medium;
c) co-culture incubation for 18 - 24 hours, preferably for 24 hours at 30 - 37 °C, preferably 30 °C;
d) cell separation by centrifugation. According to a process variant the preparation can also contain lactic acid bacteria cells;
e) preparation dehydration by drying or freeze-drying process;
f) production of dermatological preparation.

### Example 2: Study about the effect of the biomass according to the invention and Plantaricin A in wound healing

### Method

Cultured human keratinocytes have been incubated with Plantaricin A (2.5 µg/ml) or Plantaricin A containing biomass for 1 hour.

At the end of the incubation, the cells have been washed and culture medium restored.

### Wound healing

The test consists of carrying out a mechanical interruption in the continuity of the cellular monolayer in order the treatment effect in favouring or not the keratinocyte ability to migrate beyond the damage border and therefore "heal the lesion" to be evaluated.

To this end the keratinocyte monolayer, treated as above described, was incubated over additional 24 hours after stimulus application and then fixed and stained using ematoxylin/eosin staining. Images have been observed with light microscope using 5X objective.

For each image maximum cell migration limits and distance or gap there between have been detected and calculated, respectively (Fig. 9-10).

### Results

In Figure 9, results of wound healing assay are reported. After a 24 hour incubation following the interruption of cellular continuity, not stimulated keratinocyte monolayer (control) displays some cells extending from wound margins. The cellular migration is however particularly apparent when the cells are stimulated with hyaluronic acid. In this case in fact the lesion gap is narrower than for not treated cells (control), evidencing the cell migration ability for wound healing.

In order to estimate analytically cell migration ability, the wound margins have been outlined, measured and analyzed as reported in Fig.10.

Plantaricin A or Plantaricin A containing biomass incubated keratinocytes reduce the gap between the margins wound in comparison to control cell, the percentage of healing increase in comparison to control is reported in Fig. 11.

### Assay System: human reconstructed epidermis

Used epidermis model is produced by Skinethic® Laboratories, Nice (France) and is used as 0.5 cm² specimen from differentiation 17^{th} day with batch average thickness of 120 µ (corneous layer and vital epidermis).

Completely differentiated epidermis is obtained from human keratinocytes cultivated in a chemically defined medium (MCDB 153) without calf serum addition, on porous polycarbonate inert support at air-liquid interface over 17 days; at this differentiation stage the morphologic analysis shows a multilayer vital epidermis and corneous layer consisting of more than 10 compact cell layers.

### TEER DETERMINATION

Trans-epithelial electric resistance (TEER) is a direct measurement of the cutaneous barrier functionality: it reflects the tissue resistance as a whole resulting from both thickness and structure. It reflects integrity of the intercellular contacts at tight junction level, bi-lamellar lipid structure protecting from the penetration of outside substances.

TEER is an assay discriminating parameter - *Rat skin electrical resistance* (B 40) - EU validated test for the corrosiveness evaluation considering as end-points the integrity of the corneous layer and barrier function.

It is inversely proportional to TEWL as in vivo measured, which is the measure of trans-epidermal water loss: higher TEWL corresponds to higher damage of barrier function while higher TEER corresponds to lower damage of barrier function.

Over the insert 1 ml of PBS is dosed and the trans-epithelial electric resistance is measured using Millicell-ERS instrument (range 0-20 kΩ).

Various measurements have been carried out for each tissue.

Figure 5 shows TEER values referring to an average for 3 tissues, 3 determinations being carried out for each thereof.

In relation to TEER value the following properties are important: the presence of a lamellar compact structure at corneous layer level, integral tight junctions and epidermal thickness that as a whole define an efficient barrier function. Every tissue is own reference with determinations at t=0 and t= 24 hours.

Obtained results are particularly interesting, in fact it is apparent a TEER remarkable increase both in the presence of Plantaricin A and Plantaricin A containing biomass.

This increase is a directed consequence both of the epidermal thickness increase and a better compactness and integrity of the corneous layer at tight junctions level.

### Example 3: Plantaricin A: study about the role thereof for the maintenance and restoration of cutaneous barrier function

The study is based on the use of Plantaricin A obtained by *L. plantarum* DC400 (DSM 23213) and *L. rossiae* DPPMA174 (DSM 23214) co-culture in order the following goals to be obtained:
Evaluation of the biomass effects on *wound repair,* through the study of the effects on the migration and proliferation of human keratinocyte monolayer (NCTC2544) compared to a positive control (not treated cells) and a commercially available/negative known activity control. The treatments will be carried out at different concentrations of the subject Plantaricin (after determination using cytotoxicity assay) at three successive times (24, 48, 72 hours).

Analysis of the cell damage response, at considered times and concentrations, evaluating the mediator modulation like IL-8, KGF (keratinocyte growth factor), TGF-β1 (transforming growth factor-β), compared to a positive control (not treated cells) and a commercially available/negative known activity control, by means of Real-Time PCR.

### MATERIALS AND METHODS

### Cell cultures

The used cell line is a human NCTC 2544 keratinocyte cell line (Perry, V.P., Sanford, K.K., Evans, V.J., Hyatt, G.W., Earle, W.R., 1957. Establishment of clones of epithelial cells from human skin. J Natl Cancer Inst. 18 (5): 709-717) cultured in sterile flasks, incubated at 37°C in humid atmosphere at 5% CO₂ in MEM (Minimum Essential Medium) culture medium added with 10% bovine calf serum (FBS), 2 mM L-glutamine, 1% not essential amino acids, in the presence of 1% penicillin and streptomycin. Cells grow in vitro adhering to culture plate surface as a monolayer.

### STUDY ON TISSUE DAMAGE REMEDY OVER TIME BY MEANS OF WOUND HEALING

### Principle of the method:

The experiment firstly involves the development of confluent cell monolayer. Successively a cut is carried out and the generated "gap" is observed using microscope as cells gradually moving repair the damage.

This cicatrisation process (said "healing") can last from several hours to more than a day depending on cell line, wound conditions and extent.

### Experimental procedure

∘ NCTC 2544 cells are plated and cultured at 37°C, with 5% CO₂ for 24 hours.
∘ After 24 hours complete growth medium is replaced with serum free medium to avoid the serum effect on cell proliferation and cells are incubated for further 24 hours at 37°C, 5% CO₂.
∘ After 24 hours the cell monolayer is mechanically damaged by mild 200 µl pipette tip brushing tracking a ∼ 1 mm wide horizontal line. Then the monolayer is washed and after addition of substances to be tested, the plates are incubated at 37°C, 5% CO₂ for 72 hours.
∘ The effect of the substances on cell motility is evaluated using phase contrast reverse microscope, acquiring images at various opportunely selected analysis times.
∘ The damage remedy is determined measuring the clear area between the cut two migration fronts at 0, 4, 8, 12, 24, 48, 72 hours after the cut, using an image processing software (Leica application Suite).
∘ All the data for each experiment are Excel statistically processed for determinations at 0, 4, 8, 12, 24, 48 and 72 hours.

### ANALYSIS OF CELL DAMAGE RESPONSE BY EVALUATION OF IL-8, KGF, TGF-β GENE EXPRESSION USING REAL-TIME PCR

The procedure consists of 3 fundamental steps:
I. Extraction of total RNA from the cells
II. Retro-transcription in cDNA
III. Real-Time PCR

### I. Extraction of total RNA from the cells

Immortalized NCTC 2544 human keratinocyte cell line, maintained in culture flasks, incubated at 37°C in humid atmosphere at 5% CO₂ in MEM (Minimum Essential Medium) culture medium added with 10% calf serum (FBS), 2 mM glutamine, 1% not essential amino acids, in the presence of 1% penicillin and streptomycin, is used. The cell line will be scraped using a 200 µl pipette tip and treatments at different times and concentrations.

### II. RNA retro-transcription in cDNA

The process includes amplification of RNA samples extracted using *"High Capacity cDNA Reverse Transcription Kit"* (Applied Byosistem).

### III. Real-Time PCR

The process includes cDNA amplification using specific Taqman Gene assay and "TaqMan Universal PCR Master Mix with Amperase UNG 2X" kit (Applied Biosystem).

A relative type quantification to determine a possible variation of target gene expression with respect to a positive control (not treated cells) will be used by means of housekeeping gene for data normalization and data analysis according to 2^{-ΔΔCt} method.

### RESULTS

### Wound healing

The ability of co-culture deriving Plantaricin to modify the migration of human NCTC 2544 keratinocytes by wound healing assay has been evaluated.

The study concerned also the comparative evaluation of the effects of synthetic and *L. plantarum* DC400 and *L. rossiae* DPPMA174 co-culture deriving Plantaricin.

After a cut on the cellular monolayer has been carried out using a 200 µl pipette tip and the cells within the gap between two cut lines completely removed, said cells are treated with synthetic and co-culture deriving Plantaricin at the following concentrations: 0.1 - 1 - 10 µg/ml. Also positive and negative controls (connectivine at 100 µg/ml) have been duplicate tested.

Images on the same cut area have been acquired at time 0 and after 4, 8, 12, 24, 48 and 72 hours, to monitor the cell migration in the cut area.

Figures 12-16 shows the temporal analysis for the effect of co-cultured (0.1, 1 and µg/ml) (a) and synthetic Plantaricin (0.1, 1 and µg/ml) (b) on NCTC 2544 cell line migration.

Figure 12 shows the cicatrisation progression (Δµm/time) at each considered time (at 4 hour interval) for both tested actives and positive and negative controls.

During early steps of cellular cicatrisation it is not possible to detect meaningful differences at time 0 in cell migration between the controls and tested actives. As it is apparent in figure 12a, co-cultured Plantaricin at concentration of 0.1 and 1 µg/ml is not suitable to accelerate remarkably the cell migration compared to the control. The effect is already significant 4 hours after the cut and remains constant and significant up to 72 hours. Treatments with co-cultured Plantaricin at a concentration of 10 µg/ml produce instead a remedy of the tissue damage comparable to positive control, i.e. not treated cells, for all the considered time intervals.

A similar experiment carried out with a cell treatment for the same temporal period at equivalent concentrations of synthetic Plantaricin proved that also in this case the treatments with Plantaricin (synthetic) have a greater ability to accelerate the cell migration within the two cut lines in comparison to the control negative.

Particularly, also in this case, during early steps of cellular cicatrisation it is not possible to detect meaningful differences at time 0 in cell migration both for two used controls and two under study actives.

Figure 12b shows that synthetic Plantaricin at concentration of 0.1 and 10 µg/ml, respectively, produces an increase of cell migration higher than both positive and negative controls, already 4 hours after the cut and remains constant for all time intervals considered up to 72 hours. Treatments with synthetic Plantaricin at a concentration of 10 µg/ml produce instead a remedy of the tissue damage comparable to positive and negative controls. In order to comprise more completely and analyze the effects of the treatments with co-cultured and synthetic Plantaricin with respect to positive and negative controls in all the considered time intervals, we have analyzed statistically the measurements deriving from image analysis.

Figures 13 and 14 report the data as percentage of cells migrated through the two cut lines in comparison to the positive control for treatments both with co-cultured (Fig.13) and synthetic Plantaricin (Fig.14), respectively. Also the results are reported as area percentages for the two cut line and initial areas, respectively (Fig. 15-16).

Charts reported in figures 13 and 14 show the data relating to percentages of cells migrated during various considered time intervals for positive and negative controls, co-cultured (0.1-1 and 10 µg/ml) and synthetic Plantaricin (0.1-1 and 10 µg/ml), respectively. In agreement with the data deriving from image processing, after first 8 hours of treatment, negative control does not persist in cut cicatrisation activity and presents a percentage of migrated cells similar to the positive control.

As to co-cultured Plantaricin (Figure 13) in general terms it is possible to assert that, at all three used treatment concentrations, it is possible to outline a greater increment of the cell migration from time 0 up to 72 hour treatment, also at lowest concentration treatment.

In particular, treatments with co-cultured Plantaricin at concentration of 10 µg/ml shows highest migration percentage values for all considered time intervals except after 72 hour treatment, but these data are not significant with percentage values of migrated cells of 165,56%, 138.18%, 134.64%, 118.08%, 139.07% and 149.04%, respectively.

Figure 14 shows the percentage of cells migrated after treatment at various considered time intervals, at equivalents concentrations of synthetic Plantaricin.

The treatment with synthetic Plantaricin at a concentration of 0.1 µg/ml produces the highest percentage values of migrated cells for all the considered time intervals, with values from the starting time up to 72 hours of treatment of 158.30%, 115.15%, 124.32%, 141.82%, 141.91% and 128.51%, respectively. The treatment with synthetic Plantaricin at a concentration of 10 µg/ml results in a percentage of migrate cells for all the considered time intervals, compared to a treatment with same active at a concentration of 0.1 µg/ml, but at the same time results in an higher increase of the percentage of migrated cells compared to negative control after 8 up to 72 hours of treatment with a percentage increase of + 4.62%, +20.28%, +14.84% and +7.47%, respectively. On the contrary the treatment with synthetic Plantaricin at a concentration of 1 µg/ml produces a greater increase of the percentage of migrated cells compared to negative control negative only after 12 and 24 hours of treatment with percentage values of +9.86% and 15.48%, respectively.

Although the treatment of cells with the same concentrations of co-cultured and synthetic Plantaricin does not result in a percentage increase of migrated cells higher than negative control at all considered time intervals, the comparison of results from wound healing assays on human NCTC 2544 keratinocytes treated with co-cultured Plantaricin with those from synthetic Plantaricin under same conditions allowed to demonstrate that co-cultured Plantaricin has a greater effect on cell migration through the generated cut area compared to the considered negative control. TGFβ1□ gene is the most involved in the cicatrisation process.

Among all times considered for wound healing monitoring the evaluation of gene expression has been carried out after 8 hours of treatment as a confirmation of the data obtained from cicatrisation evaluation.

The obtained results outline and confirm the remarkable effect of the connectivine on the cicatrisation.

As to the data on synthetic Plantaricin is pointed out that the gene expression is remarkably increased even if it is possible to evidence that at same concentrations (1 e 10 µg/ml) co-cultured Plantaricin stimulates an higher increase of gene expression, a further confirmation of the biomass effect.

## Claims

1. Biotechnological process for the synthesis of a biomass comprising or consisting of at least one plantaricin selected from plantaricins A, K or N, or mixtures thereof and *Lactobacillus plantarum* DSM 23213 and *Lactobacillus rossiae* DSM 23214 lactic acid bacteria, said process comprising or consisting of the following steps:
a) culture propagation of *Lactobacillus plantarum* DSM 23213 and *Lactobacillus rossiae* DSM 23214 lactic acid bacteria;
b) co-inoculation of a substrate selected from the group consisting of Chemically Defined Medium, Wheat Flour Hydrolyzate, grape must, milk serum or fruit and vegetable product extracts, with aqueous suspension of lactic acid bacteria as defined in step a);
c) incubation; and, optionally,
d) centrifugation of the culture broth in order to remove the lactic acid bacteria cells.

2. Biotechnological process for the preparation of one or more plantaricins selected from plantaricins A, K or N or mixtures thereof, said process comprising or consisting of the following steps:
a) culture propagation of *Lactobacillus plantarum* DSM 23213 and *Lactobacillus rossiae* DSM 23214 lactic acid bacteria;
b) co-inoculation of a substrate selected from the group consisting of Chemically Defined Medium, Wheat Flour Hydrolyzate, grape must, milk serum or fruit and vegetable product extracts, with aqueous suspension of lactic acid bacteria as defined in step a);
c) incubation; and, optionally,
d) centrifugation of the culture broth in order to remove the lactic acid bacteria cells.

3. Process according to anyone of claims 1-2, wherein the cell density of suspension from step a) is about 9.0 Log ufc/ml for each lactic acid bacteria species and it is added to the substrate in a percentage ranging from 1 to 4% respect to the substrate volume.

4. Process according to any one of preceding claims, wherein the incubation is carried out at a temperature from 30 to 37°C, preferably 30°C, for 18 - 24 hours, preferably 18 hours.

5. Process according to any one of preceding claims wherein the centrifugation is carried out at 10.000 x g for 15 min a 4°C.

6. Process according to any one of preceding claims, further comprising the step e) for dehydration of the supernatant obtained in step d) by means drying or freeze-drying.

7. Biomass obtainable or obtained by means of the process as defined in any one of claims 1-6, said biomass comprising or consisting of at least one plantaricin selected from plantaricins A, K or N or mixtures thereof and *Lactobacillus plantarum* DSM 23213 and *Lactobacillus rossiae* DSM 23214 lactic acid bacteria.

8. Pharmaceutical composition comprising or consisting of the biomass as defined in claim 7, as an active principle, in association with one or more pharmaceutically acceptable excipients and/or adjuvants.

9. Cosmetic composition comprising or consisting of the biomass as defined in claim 7, as an active principle, in association with one or more pharmaceutically acceptable excipients and/or adjuvants.

10. Use of biomass as defined in claim 7 or composition as defined in claim 8 for the preparation of a medicament for the treatment or the prevention of the barrier function damage of epidermis or intestinal wall.

11. Use of biomass as defined in claim 7 or composition as defined in claim 8 for the preparation of medicament for wound healing.

12. *Lactobacillus plantarum* DSM 23213 or *Lactobacillus rossiae* DSM 23214 lactic acid bacteria or mixture thereof.

13. Use of one or more plantaricins selected from plantaricins A, K or N, preferably A, for the preparation of a medicament for the treatment or the prevention of the barrier function damage of epidermis or intestinal wall.

14. Use of one or more plantaricins selected from plantaricins A, K or N, preferably A, for the preparation of a medicament for wound healing.

## Patentansprüche

1. Biotechnisches Verfahren zur Synthese einer Biomasse, welche mindestens ein Plantaricin, das aus den Plantaricinen A, K oder N oder Mischungen davon ausgewählt ist, und die Milchsäurebakterien *Lactobacillus plantarum* DSM 23213 und *Lactobacillus rossiae* DSM 23214 umfasst oder daraus besteht, wobei das Verfahren die folgenden Schritte umfasst oder daraus besteht:
a) Vermehrung der Milchsäurebakterien *Lactobacillus plantarum* DSM 23213 und *Lactobacillus rossiae* DSM 23214 in Kultur;
b) gemeinsame Beimpfung eines Substrats, das aus der Gruppe ausgewählt ist, welche aus chemisch definiertem Medium, Weizenmehlhydrolysat, Traubenmost, Milchserum oder Extrakten von Frucht- und Gemüseprodukten besteht, mit einer wässrigen Suspension von Milchsäurebakterien wie in Schritt a) definiert;
c) Inkubation; und, gegebenenfalls,
d) Zentrifugation der Kulturbouillon, um die Zellen der Milchsäurebakterien zu entfernen.

2. Biotechnisches Verfahren zur Herstellung von einem oder mehreren Plantaricinen, das bzw. die aus Plantaricinen A, K oder N oder Mischungen davon ausgewählt ist/sind, wobei das Verfahren die folgenden Schritte umfasst oder daraus besteht:
a) Vermehrung der Milchsäurebakterien *Lactobacillus plantarum* DSM 23213 *und Lactobacillus rossiae* DSM 23214 in Kultur;
b) gemeinsame Beimpfung eines Substrats, das aus der Gruppe ausgewählt ist, welche aus chemisch definiertem Medium, Weizenmehlhydrolysat, Traubenmost, Milchserum oder Extrakten von Frucht- und Gemüseprodukten besteht, mit einer wässrigen Suspension von Milchsäurebakterien wie in Schritt a) definiert;
c) Inkubation; und, gegebenenfalls,
d) Zentrifugation der Kulturbouillon, um die Zellen der Milchsäurebakterien zu entfernen.

3. Verfahren nach irgendeinem der Ansprüche 1-2, wobei die Zelldichte der Suspension aus Schritt a) etwa 9,0 Log CFU/ml für jede Milchsäurebakterienspezies beträgt und diese dem Substrat in einem Prozentsatz zugegeben wird, der im Bereich von 1 bis 4 %, bezogen auf das Substratvolumen, liegt.

4. Verfahren nach irgendeinem der vorhergehenden Ansprüche, wobei die Inkubation bei einer Temperatur von 30 bis 37 °C, vorzugsweise 30 °C, für 18-24 Stunden, vorzugsweise 18 Stunden, durchgeführt wird.

5. Verfahren nach irgendeinem der vorhergehenden Ansprüche, wobei die Zentrifugation bei 10.000 x g für 15 min bei 4° C durchgeführt wird.

6. Verfahren nach irgendeinem der vorhergehenden Ansprüche, ferner umfassend den Schritt e) zur Entwässerung des in Schritt d) erhaltenen Überstands mittels Trocknung oder Gefriertrocknung.

7. Biomasse, erhältlich oder erhalten mit dem in irgendeinem der Ansprüche 1-6 definierten Verfahren, wobei die Biomasse mindestens ein Plantaricin, das aus den Plantaricinen A, K oder N oder Mischungen davon ausgewählt ist, und die Milchsäurebakterien *Lactobacillus plantarum* DSM 23213 und *Lactobacillus rossiae* DSM 23214 umfasst oder daraus besteht.

8. Pharmazeutische Zusammensetzung, welche die Biomasse wie in Anspruch 7 definiert als Wirkstoff in Assoziation mit einem oder mehreren pharmazeutisch annehmbaren Exzipienten und/oder Adjuvanzien umfasst oder daraus besteht.

9. Kosmetische Zusammensetzung, welche die Biomasse wie in Anspruch 7 definiert als Wirkstoff in Assoziation mit einem oder mehreren pharmazeutisch annehmbaren Exzipienten und/oder Adjuvanzien umfasst oder daraus besteht.

10. Verwendung der Biomasse wie in Anspruch 7 definiert oder der Zusammensetzung wie in Anspruch 8 definiert zur Herstellung eines Medikaments für die Behandlung oder Verhütung einer Beeinträchtigung der Barrierefunktion von Epidermis oder einer Darmwand.

11. Verwendung der Biomasse wie in Anspruch 7 definiert oder der Zusammensetzung wie in Anspruch 8 definiert zur Herstellung eines Medikaments für die Wundheilung.

12. Milchsäurebakterien *Lactobacillus plantarum* DSM 23213 und *Lactobacillus rossiae* DSM 23214 oder eine Mischung davon.

13. Verwendung von einem oder mehreren Plantaricin(en), das bzw. die aus den Plantaricinen A, K oder N ausgewählt sind, vorzugsweise von A, zur Herstellung eines Medikaments für die Behandlung oder Verhütung einer Beeinträchtigung der Barrierefunktion von Epidermis oder einer Darmwand.

14. Verwendung von einem oder mehreren Plantaricin(en), das bzw. die aus den Plantaricinen A, K oder N ausgewählt sind, vorzugsweise von A, zur Herstellung eines Medikaments für die Wundheilung.

## Revendications

1. Procédé biotechnologique pour la synthèse d'une biomasse comprenant ou constituée d'au moins une plantaricine choisie parmi les plantaricines A, K ou N, ou leurs mélanges et les bactéries lactiques *Lactobacillus plantarum* DSM 23213 et *Lactobacillus rossiae* DSM 23214, ledit procédé comprenant ou étant constitué des étapes suivantes :
a) la propagation de la culture des bactéries lactiques *Lactobacillus plantarum* DSM 23213 et *Lactobacillus rossiae* DSM 23214 ;
b) la co-inoculation d'un substrat choisi dans le groupe constitué du milieu chimiquement défini, l'hydrolysat de farine de blé, le moût de raisin, le lactosérum ou des extraits de produits de fruits et de légumes, avec une suspension aqueuse des bactéries lactiques telles que définies dans l'étape a) ;
c) l'incubation ; et, éventuellement,
d) la centrifugation du bouillon de culture afin d'éliminer les cellules des bactéries lactiques.

2. Procédé biotechnologique pour la préparation d'une ou de plusieurs plantaricines choisies parmi des plantaricines A, K ou N ou leurs mélanges, ledit procédé comprenant ou étant constitué des étapes suivantes :
a) la propagation de la culture des bactéries lactiques *Lactobacillus plantarum* DSM 23213 et *Lactobacillus rossiae* DSM 23214 ;
b) la co-inoculation d'un substrat choisi dans le groupe constitué du milieu chimiquement défini, l'hydrolysat de farine de blé, le moût de raisin, le lactosérum ou des extraits de produits de fruits et de légumes, avec une suspension aqueuse des bactéries lactiques telles que définies dans l'étape a) ;
c) l'incubation ; et, éventuellement,
d) la centrifugation du bouillon de culture afin d'éliminer les cellules des bactéries lactiques.

3. Procédé selon l'une quelconque des revendications 1 et 2, dans lequel la densité cellulaire de la suspension issue de l'étape a) est d'environ 9,0 Log ufc/ml pour chaque espèce de bactéries lactiques et elle est ajoutée au substrat dans un pourcentage situé dans la plage de 1 à 4 % par rapport au volume du substrat.

4. Procédé selon l'une quelconque des revendications précédentes, dans lequel l'incubation est réalisée à une température de 30 à 37 °C, de préférence 30 °C, pendant 18 à 24 heures, de préférence 18 heures.

5. Procédé selon l'une quelconque des revendications précédentes dans lequel la centrifugation est réalisée à 10 000 x g pendant 15 min à 4 °C.

6. Procédé selon l'une quelconque des revendications précédentes, comprenant en outre l'étape e) pour la déshydratation du surnageant obtenu dans l'étape d) au moyen d'un séchage ou d'une cryodessiccation.

7. Biomasse pouvant être obtenue ou obtenue au moyen du procédé tel que défini dans l'une quelconque des revendications 1 à 6, ladite biomasse comprenant ou étant constituée d'au moins une plantaricine choisie parmi les plantaricines A, K ou N, ou leurs mélanges et les bactéries lactiques *Lactobacillus plantarum* DSM 23213 et *Lactobacillus rossiae* DSM 23214.

8. Composition pharmaceutique comprenant ou étant constituée de la biomasse telle que définie dans la revendication 7, en tant que principe actif, en association avec un ou plusieurs excipients et/ou adjuvants pharmaceutiquement acceptables.

9. Composition cosmétique comprenant ou étant constituée de la biomasse telle que définie dans la revendication 7, en tant que principe actif, en association avec un ou plusieurs excipients et/ou adjuvants pharmaceutiquement acceptables.

10. Utilisation de la biomasse telle que définie dans la revendication 7 ou de la composition telle que définie dans la revendication 8 pour la préparation d'un médicament destiné au traitement ou à la prévention d'un endommagement de la fonction de barrière de l'épiderme ou de la paroi intestinale.

11. Utilisation de la biomasse telle que définie dans la revendication 7 ou de la composition telle que définie dans la revendication 8 pour la préparation d'un médicament destiné à la cicatrisation.

12. Bactéries lactiques *Lactobacillus plantarum* DSM 23213 et *Lactobacillus rossiae* DSM 23214 ou leur mélange.

13. Utilisation d'une ou de plusieurs plantaricines choisies parmi les plantaricines A, K ou N, de préférence A, pour la préparation d'un médicament destiné au traitement ou à la prévention d'un endommagement de la fonction de barrière de l'épiderme ou de la paroi intestinale.

14. Utilisation d'une ou de plusieurs plantaricines choisies parmi les plantaricines A, K ou N, de préférence A, pour la préparation d'un médicament destiné à la cicatrisation.


### SEQUENCE LISTING

<110> Giuliani SpA
<120> Process for the preparation of a biomass comprising plantaricin
   and uses thereof in medical field
<130> PCT28086
<150> RM2010A000004
   <151> 2010-01-12
<160> 1
<170> PatentIn version 3.5
<210> 1
   <211> 26
   <212> PRT
   <213> Lactobacillus plantarum
<400> 1
   Lys Ser Ser Ala Tyr Ser Leu Gln Met Gly Ala Thr Ala Ile Lys Gln
   1               5                   10                  15
   Val Lys Lys Leu Phe Lys Lys Trp Gly Trp
               20                  25